(19) Europäisches Patentamt
European Patent Office
Office européen des brevets

(11) **EP 4 478 308 A1**

(12) **EUROPEAN PATENT APPLICATION**
published in accordance with Art. 153(4) EPC

(43) Date of publication:
**18.12.2024 Bulletin 2024/51**

(21) Application number: **22934652.3**

(22) Date of filing: **19.08.2022**

(51) International Patent Classification (IPC):
**G06V 40/70** (2022.01)    **G06V 40/16** (2022.01)

(52) Cooperative Patent Classification (CPC):
**G06V 40/16; G06V 40/70**

(86) International application number:
**PCT/CN2022/113755**

(87) International publication number:
**WO 2023/184832 (05.10.2023 Gazette 2023/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 31.03.2022  CN 202210344726
31.03.2022  CN 202210344718
31.03.2022  CN 202210346427
31.03.2022  CN 202210346417

(71) Applicant: **Shanghai Sensetime Intelligent
Technology Co.,
Ltd.
Shanghai 200233 (CN)**

(72) Inventors:
• **HE, Yukang**
**Shanghai 200233 (CN)**
• **GAO, Yong**
**Shanghai 200233 (CN)**
• **MAO, Ningyuan**
**Shanghai 200233 (CN)**
• **XU, Liang**
**Shanghai 200233 (CN)**

(74) Representative: **Gevers Patents**
**De Kleetlaan 7A**
**1831 Diegem (BE)**

(54) **PHYSIOLOGICAL STATE DETECTION METHOD AND APPARATUS, ELECTRONIC DEVICE, STORAGE MEDIUM, AND PROGRAM**

(57)    Embodiments of the present invention provide a physiological state detection method and apparatus, an electronic device, a storage medium, and a program. The method comprises: acquiring a video stream collected by a photographing device; extracting multiple frames of face images of a target object from multiple frames of images in the video stream; determining at least one smooth area in the face images; performing weighting processing or area screening on the at least one smooth area on the basis of area attribute information, so as to obtain information to be extracted that is related to a physiological state; and performing physiological state information extraction on the information to be extracted, so as to obtain a physiological state detection result of the target object. According to the embodiments of the present invention, the physiological state detection is implemented on the basis of an imaging processing mode, real-time measurement can be performed anytime and anywhere, the practicability is better, and the accuracy of measurement can be improved by means of the constraint of preset conditions.

FIG. 1

**Description**

CROSS-REFERENCE TO RELATED APPLICATIONS

Embodiments of the present application are based on and claim priority to four Chinese Patent Applications filed on March 31, 2022 by Shanghai Sense Time Lingang Intelligent Technology Co., the four Chinese Patent Applications include: Chinese Patent Application No. 202210344726.1, titled "PHYSIOLOGICAL STATE DETECTION METHOD AND APPARATUS, ELECTRONIC DEVICE, AND STORAGE MEDIUM"; Chinese Patent Application No. 202210344718.7, titled "PHYSIOLOGICAL STATE DETECTION METHOD AND APPARATUS, ELECTRONIC DEVICE, AND STORAGE MEDIUM"; Chinese Patent Application No. 202210346427.1, titled "BLOOD PRESSURE MEASUREMENT METHOD AND APPARATUS, ELECTRONIC DEVICE, AND STORAGE MEDIUM"; and Chinese Patent Application No. 202210346417.8, titled "PHYSIOLOGICAL STATE DETECTION METHOD AND APPARATUS, ELECTRONIC DEVICE, AND STORAGE MEDIUM", the entire contents of which are incorporated herein by reference in their entirety.

TECHNICAL FIELD

The present application relates to the field of computer technologies, and in particular, to a physiological state detection method and apparatus, an electronic device, a storage medium, and a program.

BACKGROUND

Accurate physiological state data is the basis for analyzing human variability. Therefore, it is of great significance to detect the physiological state. Taking a safe driving scenario as an example, effective physiological state detection may help understand the physiological state of occupants in a vehicle, thereby providing supportive decisions for safe driving. In the related art, physiological state detection mainly relies on specialized detection devices, such as a blood pressure meter, a heart rate meter, a blood oxygen meter, or the like. In addition, physiological state measurement may further be implemented with wearable devices integrated with related sensing components, such as a smart watch, a smart bracelet, or the like.

It can be known that in the aforementioned detection solution, specialized instruments are required for contact measurements, which brings inconvenience to the detection and thus can not well satisfy requirements of, for example, a safe driving scenario.

SUMMARY

Embodiments of the present application at least provide a physiological state detection method and apparatus, an electronic device, a storage medium, and a program.

An embodiment of the present application provides a physiological state detection method including the following operations. A video stream collected by a camera device is obtained. Multiple frames of face images of a target object are extracted from multiple frames of images in the video stream. At least one smooth region in the face image is determined. Weighted processing or region filtering is performed on the at least one smooth region based on region attribute information, to obtain to-be-extracted information associated with a physiological state. Physiological state information extraction is performed on the to-be-extracted information to obtain a physiological state detection result of the target object.

An embodiment of the present application further provides a physiological state detection apparatus including an obtaining part, an extraction part, a determination part, a processing part and a detection part. The obtaining part is configured to obtain a video stream collected by a camera device. The extraction part is configured to extract multiple frames of face images of a target object from multiple frames of images in the video stream. The determination part is configured to determine at least one smooth region in the face image. The processing part is configured to perform weighted processing or region filtering on the at least one smooth region based on region attribute information, to obtain to-be-extracted information associated with a physiological state. The detection part is configured to perform physiological state information extraction on the to-be-extracted information to obtain a physiological state detection result of the target object.

An embodiment of the present application further provides an electronic device including a processor, a memory and a bus. The memory has stored thereon machine-readable instructions executable by the processor. The processor and the memory communicate through the bus when the electronic device is in operation. The machine-readable instructions, when executed by the processor, perform the aforementioned physiological state detection method.

An embodiment of the present application further provides a computer-readable storage medium having stored thereon a computer program that when run by a processor, performs the aforementioned physiological state detection method.

An embodiment of the present application further provides a computer program including computer-readable codes. In case that the computer-readable codes are run in an electronic device, a processor of the electronic device performs the aforementioned physiological state detection method.

With the physiological state detection method and apparatus, the electronic device, the storage medium, and the program provided by the embodiments of the present application, in case that the video stream is obtained, multiple frames of face images of the target object may be extracted from the video stream, and at least one smooth region in each frame of face image may be determined. The determined at least one smooth region may then be weighted or filtered based on the region attribute information of the smooth region, such that the physiological state detection result of the target object may be obtained by performing processing based on the weighted or filtered result. Compared with the related art in which there is a problem of measurement inconvenience as specialized instruments are required for contact measurements, in the present application, the physiological state detection is implemented based on an image processing manner, and the measurement may be performed anytime and anywhere in real time, which has a better practicality. In this way, accuracy and flexibility of the measurement may be improved.

In order to make the aforementioned purpose, features and advantages of the present application more obvious and understandable, preferred embodiments in combination with drawings are illustrated in detail below.

BRIEF DESCRIPTION OF THE DRAWINGS

In order to illustrate the technical solutions of the embodiments of the present application more clearly, drawings used in the embodiments of the present application are illustrated below. The drawings, which are incorporated into and constitute a part of the description, illustrate embodiments consistent with the present application, and are used together with the description to illustrate the technical solutions of the present application.

FIG. 1 illustrates a schematic flowchart of a first physiological state detection method provided by an embodiment of the present application.

FIG. 2A illustrates a schematic diagram of face feature points that may be extracted from a face image captured by a camera.

FIG. 2B illustrates a schematic flowchart of a second physiological state detection method provided by an embodiment of the present application.

FIG. 2C illustrates a schematic flowchart of a third physiological state detection method provided by an embodiment of the present application.

FIG. 2D illustrates a schematic flowchart of a fourth physiological state detection method provided by an embodiment of the present application.

FIG. 2E illustrates a schematic flowchart of a fifth physiological state detection method provided by an embodiment of the present application.

FIG. 3 illustrates a schematic diagram of a physiological state detection apparatus provided by an embodiment of the present application.

FIG. 4 illustrates a schematic diagram of an electronic device provided by an embodiment of the present application.

DETAILED DESCRIPTION

In order to make the purpose, technical solutions and advantages of the embodiments of the present application more clearly, the technical solutions in the embodiments of the present application will be described clearly and completely in the following in combination with the drawings in the embodiments of the present application. It is apparent that the described embodiments are only a part of the embodiments of the present application and not all of the embodiments. Generally, components of the embodiments of the present application described and illustrated in the drawings herein may be arranged and designed in various different configurations. Accordingly, the following detailed description of the embodiments of the present application provided in the drawings is not intended to limit the scope of protection of the present application, but only indicates selected embodiments of the present application. Based on the embodiments of the present application, all other embodiments obtained by those of ordinary skill in the art without creative effort fall within the scope of protection of the present application.

It is to be noted that similar numbers and letters indicate similar items in the drawings below. Therefore, once an item is defined in a drawing, it is not necessary to further define and explain the item in subsequent drawings.

The term "and/or" herein is only a description of an association relationship, indicating that there may be three kinds of relationships. For example, "A and/or B" may indicate three conditions, i.e., independent existence of A, existence of both A and B, and independent existence of B. In addition, the term "at least one" herein indicates any one of multiple ones or any combination of at least two of multiple ones. For example, "including at least one of A, B or C" may indicate including any one or more elements selected from a set that consists of A, B and C.

It is found through research that in the related art, physiological state detection mainly relies on specialized detection devices, such as a blood pressure meter, a heart rate meter, a blood oxygen meter, or the like. In addition, physiological state measurement may further be implemented with wearable devices integrated with related sensing components, such as a smart watch, a smart bracelet, or the like. It can be known that in the aforementioned detection solution, specialized instruments are required for contact measurements, which brings inconvenience to the detection and thus can not well satisfy requirements of, for example, a safe driving scenario.

In order to solve the aforementioned problems, in the related art, a solution of contactless physiological state detection, i.e., remote Photoplethysmographic (rPPG), is provided. Such method may complete the detection with the currently widely used cell phone terminal having a camera without additional hardware costs, and is very convenient to use. The current bottleneck of the rPPG method is that the detection accuracy is inferior to that of some specialized detection devices, and furthermore, it is susceptible to external light. In addition, physiological characteristics detection using the rPPG method requires the detected object to keep stationary for a period and can only be used for active detection.

In the conventional rPPG-based physiological characteristics detection, it is necessary to select a Region of Interest (ROI). In an actual image generated by the camera, the picture may be too dark or too bright. A single ROI region is usually a fixed range of face, and it is easy to select a region with poor exposure for signal extraction. Alternatively, in an actual image generated by the camera, since there is an intersection angle between the face and the optical axis of the camera, the pixel area in the image, which is occupied by the generated image of the ROI region, may be too small, leading to less amount of information of the ROI region, and thus it is difficult to obtain an ideal detection result.

Based on the aforementioned research, an embodiment of the present application provides a solution for implementing physiological state detection through limits of preset conditions, such as selecting an optimal ROI region based on image brightness, or weighting based on an region area, or weighting based on the region area and region brightness, such that a semaphore closer to the actual situation is obtained and the detection accuracy is improved.

To facilitate understanding of the embodiment of the present application, firstly, a physiological state detection method disclosed by an embodiment of the present application is introduced in detail. The execution subject of the physiological state detection method provided by the embodiment of the present application is generally an electronic device having a certain computing capability. The electronic device includes, for example, a terminal device, a server, or other processing devices. The terminal device may be User Equipment (UE), a mobile device, a user terminal, a cellular phone, a cordless phone, a Personal Digital Assistant (PDA), a handheld device, an on-board device, a wearable device, or the like. In some embodiments of the present application, the physiological state detection method may be implemented by means of a processor calling computer-readable instructions stored in a memory.

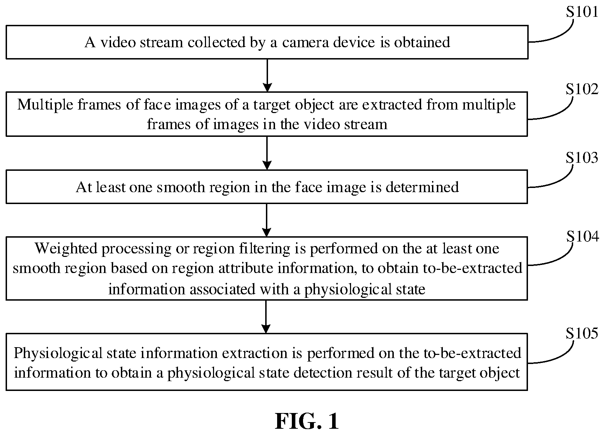

FIG. 1 is a flowchart of a physiological state detection method provided by an embodiment of the present application. Referring to FIG. 1, the method includes operations S101 to S105.

In operation S101, a video stream collected by a camera device is obtained.

In operation S102, multiple frames of face images of a target object are extracted from multiple frames of images in the video stream.

In operation S103, at least one smooth region in the face image is determined.

In operation S104, weighted processing or region filtering is performed on the at least one smooth region based on region attribute information, to obtain to-be-extracted information associated with a physiological state.

In operation S105, physiological state information extraction is performed on the to-be-extracted information to obtain a physiological state detection result of the target object.

The physiological state detection method in the embodiment of the present application may be applied to the field of automobiles in which physiological state detection is required. The embodiment of the present application may implement physiological state detection for a human body in a vehicle cabin environment. Furthermore, based on the physiological state detection method provided by the embodiment of the present application, in case that the physiological state detection result associated with the target object in the vehicle cabin environment is obtained, it is possible to know whether the target object is in an abnormal physical condition timely, and to alert or provide assistance timely in case that an abnormal physical condition occurs. In this way, it is possible to provide more sufficient realistic possibilities for safe driving.

In addition, the embodiment of the present application may be further applied to any other related fields in which physiological state detection is required, such as medical treatment, home life, or the like. Taking the wide application of the field of automobile driving into account, the following examples and illustration are mostly in the field of automobiles. Furthermore, the video stream in the embodiment of the present application may be collected by a camera device (such as an in-car fixed camera in an automobile scenario), or collected by a built-in camera of the user terminal, or collected in other manners.

In order to implement the physiological state detection for a specific target object, the installation position of the related camera may be preset based on the specific target object. For example, in order to implement the physiological state detection for a driver in a vehicle, the camera may be installed at a position where the shooting range covers the driving region, for example, inside the A-pillar, on the console, or at the position of the steering wheel of the vehicle. Furthermore, for example, in order to implement the physiological state detection for occupants with multiple riding attributes including the driver and passengers in the vehicle, the camera may be installed at a position where the shooting range can cover multiple seating regions in the vehicle cabin, such as the interior rearview mirror, the top trim, the reading lamp, or the like.

In a practical application in the field of automobiles, collection of the video stream of the related driving region may further be implemented by an in-vehicle image collection apparatus included in a Driver Monitoring System (DMS). Alternatively, collection of the video stream of the related riding region may be implemented by an in-vehicle image collection apparatus included in an Occupant Monitoring System (OMS).

It is taken into account that changes of the skin color and brightness, which are generated by the flow of blood vessels of the face, may reflect physiological states such as heartbeat and respiration. Firstly, face detection may be performed on multiple frames of images in the video stream to extract multiple frames of face images of the target object in the vehicle cabin, and then extraction of physiological state information may be implemented for the face images.

The target object is a to-be-measured object of the physiological state detection for the human body, which may be a person object that stays at a specified position, or a person object having a specific identity attribute. For example, in the field of automobiles, the target object may be an object having a specific riding attribute, such as the driver, or the occupant on the front passenger seat. Alternatively, the target object may be an object whose identity is pre-registered using face information, such as a vehicle owner registered through an application. Alternatively, the target object may further be any occupant in the vehicle, and it is possible to locate at least one occupant by performing face detection on the video stream in the vehicle cabin and to take the detected one or more occupants as the target object.

In the process of performing the face detection, there may be a case where faces of multiple objects appear on a single frame of image. In some scenarios, it is possible to select to perform the physiological state detection on an occupant at a certain riding position, i.e., the occupant at the riding position is taken as the target object. To implement the physiological state detection for the target object in the vehicle cabin, multiple frames of face images of the target object may be determined from the detected face images according to the face detection result of the multiple frames of images and the specified riding position. The specified riding position is used to indicate the position of the to-be-measured target object.

The camera for collecting the video stream in the vehicle cabin is at a fixed relative position in the interior space of the vehicle. According to the position of the camera, the images collected by the camera may be divided according to the seating region. For example, a 5-seat private car may be divided into an image region corresponding to the driver's seat, an image region corresponding to the front passenger's seat, an image region corresponding to the left rear seat, an image region corresponding to the right rear seat, and an image region corresponding to the middle rear seat. According to the position of the face of each occupant in the image and the coordinate range of each image region, the image region into which the face of each occupant falls may be determined, and then the occupant object at the designated riding position may be determined as the target object.

In a practical application, the OMS generally takes images of the entire vehicle cabin space and may capture multiple people. The to-be-measured target object in the vehicle cabin space may be specified by manually selecting "front riding seat" and "rear riding seat", and at this time, the embodiment of the present application may measure the face in the corresponding region of the image. The DMS captures the main driving region, and in case that only the driver is included in the captured object, it is not necessary to specify an object.

In some embodiments of the present application, physiological states, such as the heart rate, the respiratory rate, the blood oxygen, the blood pressure, or the like, often require a certain duration of monitoring before they can be evaluated. Therefore, in the embodiment of the present application, image change information, which corresponds to multiple frames of face images in the video stream lasting for a certain period, is adopted to implement the extraction of the physiological state information. In this way, the extracted physiological state detection result satisfies the requirements of actual scenarios better.

In some embodiments of the present application, in the process of performing image change information analysis based on a face image, there is a problem that the detection can not be continued due to loss of the ROI region (i.e., corresponding to the smooth region in the physiological state detection method provided by the embodiment of the present application), which is caused by, for example, face rotation, occlusion, or external light. Thus, the embodiment of the present application may use multiple smooth regions in the face image for detection. In this way, even if one of the smooth regions is lost, in case that other smooth regions perform well, detection may be continued, making usage scenarios more extensive.

In some embodiments, it is taken into account that there is a difference in the pixel areas occupied by the generated images of different smooth regions due to the intersection angle between the face and the optical axis of the camera. Therefore, in the embodiment of the present application, a semaphore closer to the actual situation may be further obtained by the selected multiple smooth regions and by weighting the pixel areas of the smooth regions in the picture, and thus the detection accuracy may be improved.

In some embodiments, it is further taken into account that there is a difference in the pixel areas occupied by the generated images of different smooth regions and a difference in the corresponding pixel brightness due to the intersection angle between the face and the optical axis of the camera. Therefore, a semaphore closer to the actual situation may be obtained by weighting the pixel areas of the smooth regions in the picture and the corresponding pixel brightness, and thus the detection accuracy may be improved.

At least one smooth region may be determined for each frame of face image, and the smooth region may be determined by filtering one or more face smooth sub-regions in the corresponding face image. The face smooth sub-region may be a smooth connected region. The connected region may be required to be a specified shape such as a rectangle, a circle, an ellipse, or the like, which may be located by key points of the face. Each connected region does not include non-smooth features of the face such as the eyes, the nose, the mouth, the eyebrows, or the like. To some extent, the connected region has a more uniform reflectance, thus more effective changes of the skin color and brightness generated by the flow of blood vessels of the face may be captured, and then more accurate physiological state detection may be implemented.

In case that multiple face smooth sub-regions of each frame of face image are determined, with the physiological state detection method provided by the embodiment of the present application, firstly, the at least one smooth region may be determined based on a preset condition that is set associated with the image brightness, and then, the physiological state information may be extracted based on the image change information corresponding to the image information of the at least one smooth region. The extracted physiological state detection result may be a detection result including at least one of the heart rate, the respiratory rate, the blood oxygen, the blood pressure, or the like.

In the embodiment of the present application, the optimal ROI region may be selected as the smooth region according to the image brightness of multiple face smooth sub-regions, and thereby Photoplethysmography (PPG) signal extraction may be performed on the smooth region, such that the detection accuracy of the rPPG method and the anti-interference capability in the detection are improved.

The preset condition that is set associated with the image brightness may be a condition of filtering out a related face smooth sub-region which is obviously overexposed or obviously too dark. The reason is that the image brightness corresponding to an obviously overexposed face smooth sub-region is abnormally high, and the PPG signal extraction performed in such case is not accurate enough, thereby leading to a low detection accuracy of the finally obtained physiological state detection result. Similarly, for an obviously too dark face smooth sub-region, the image brightness is abnormally low, which also leads to a low detection accuracy.

In some embodiments of the present application, firstly, face feature point extraction may be performed on the face image, and then, the at least one smooth region in the face image may be determined based on the extracted face feature points. That is, the aforementioned operation S103 may be implemented by the following process.

In a first operation, face feature point extraction is performed on the face image to obtain positions of multiple preset key feature points in the face image.

In a second operation, at least one smooth region in the face image corresponding to the multiple preset key feature points is determined based on the positions of the multiple preset key feature points.

The aforementioned process of extracting the face feature points may be implemented by a face key point detection algorithm. For example, face feature points associated with a standard face image may be preset. The standard face may be a face image which includes the five senses and is taken in front of the camera. In this way, in the process of performing the face feature point extraction on the face image of the target object extracted from each frame of image, each face feature point may be determined based on the comparison between the extracted face image of the target object and the standard face image. For example, the face feature points may be related feature points with obvious face characteristics, such as eyebrow feature points, nose bridge feature points, nose tip feature points, cheek feature points, mouth corner feature points, or the like.

In the embodiment of the present application, one or more smooth regions in the face image may be determined based on positions of multiple preset key feature points in the face image. The smooth region may be a rectangular region, and may further be other regions with a connected shape, which is not limited by the embodiment of the present application. The subsequent illustration is mostly with examples of rectangular regions.

In a practical application, the smooth region may be a forehead smooth region determined based on the eyebrow feature points; a left upper cheek smooth region and a right upper cheek smooth region determined based on the cheek feature points, the nose bridge feature points and the nose tip feature points; or a left lower cheek smooth region and a right lower cheek smooth region determined based on the cheek feature points, the nose tip feature points and the mouth corner feature points.

In some embodiments of the present application, without region occlusion, the aforementioned five regions (the forehead smooth region, the left upper cheek smooth region, the right upper cheek smooth region, the left lower cheek smooth region and the right lower cheek smooth region) may be extracted simultaneously from a single frame of face image.

Furthermore, in case of region occlusion, the region that may be actually extracted from a single frame of face image may be determined according to the actual situation. In the embodiment of the present application, the occluded smooth region may be determined according to the following operations.

In a first operation, in case that a preset key feature point is missing among the extracted face feature points, the face sub-region corresponding to the missing preset key feature point is determined as a missing region, and other face sub-regions other than the missing region among the predefined multiple face sub-regions are determined as non-missing regions.

In a second operation, a boundary of a non-missing region is determined according to the positions of the preset key feature points corresponding to the non-missing region among the extracted face feature points, such that the at least one smooth region is determined.

In some embodiments of the present application, the preset key feature point being missing among the extracted face feature points indicates that the corresponding face sub-region is occluded, and the face sub-regions corresponding to other face key points that are not missing may be determined as the non-missing regions. In case that the boundary of the non-missing region is determined based on the positions of the preset key feature points corresponding to the non-missing region, the smooth region is determined. It can be known that even if occlusion occurs, the embodiment of the present application may extract the smooth region more accurately according to the aforementioned method.

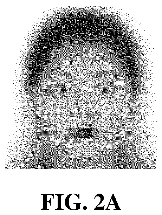

FIG. 2A is a schematic diagram of face feature points that may be extracted from a face image captured by a camera. As shown in FIG. 2A, totally, 106 feature points are extracted from the face. Five smooth regions may be filtered based on coordinate information of the face feature points. Referring to FIG. 2A, regarding region 1, a rectangular ROI of region 1 may be constructed by two feature points of the eyebrows on both sides. Region 2 is a cheek region on the left side, and a rectangular ROI of region 2 may be constructed by the positions of the feature points on the left edge of the face, the nose bridge feature points and the left eye feature points. Region 3 is a cheek region on the right side, and a rectangular ROI of region 3 may be constructed by the positions of the feature points on the right edge of the face, the nose bridge feature points and the right eye feature points. Region 4 is another cheek region on the left side, and a rectangular ROI of region 4 may be constructed by the positions of the feature points on the left edge of the face, the left nose wing feature points and the left mouth corner feature points. Region 5 is another cheek region on the right side, and a rectangular ROI of region 5 may be constructed by the positions of the feature points on the right edge of the face, the right nose wing feature points and the right mouth corner feature points.

In the process of performing the face feature point extraction, the embodiment of the present application may also be implemented in combination with a face pose. Firstly, the face pose of the target object may be obtained according to the face image detection, and then the face feature point extraction may be performed on the face image according to the face pose.

The aforementioned face pose may be determined based on a pre-trained face pose detection network. The face pose detection network may be a neural network of any structure, such as a multilayer convolutional neural network, which may learn the correspondences between the face image samples and the correspondingly annotated face poses. The face pose that may be annotated includes information such as the rotation direction of the head relative to the camera, the rotation angle, or the like. In this way, after the face image is inputted to the trained face pose detection network, the face pose of the related face may be determined, for example, a face tilted 45° to the left. In such case, the extraction of the related preset key feature points may be performed according to a standard left tilted face image in case of the face tilted 45° to the left, which is easy to operate.

In the embodiment of the present application, for different face poses, the areas of different face sub-regions presented in the image may be different, and the areas of the corresponding smooth regions may also be different. Still taking the face tilted 45° to the left as an example, in such case, the area of the left cheek region in the image is small or even almost invisible, and the area of the corresponding smooth region is close to 0.

The aforementioned region attribute information may be information associated with at least one of the attributes of the smooth region, such as the brightness, the area, the position, or the like. In the embodiment of the present application, the weight of each smooth region may be calculated or filter processing may be performed according to the aforementioned region attributes of the smooth region, and the image information of the smooth region, which is associated with the physiological state, is obtained as the to-be-extracted information after the weighting or filter processing. Alternatively, after the weight is calculated, a time-domain signal extraction operation is performed, the time-domain signal corresponding to each smooth region is weighted, and the obtained signal is taken as the to-be-extracted information associated with the physiological state.

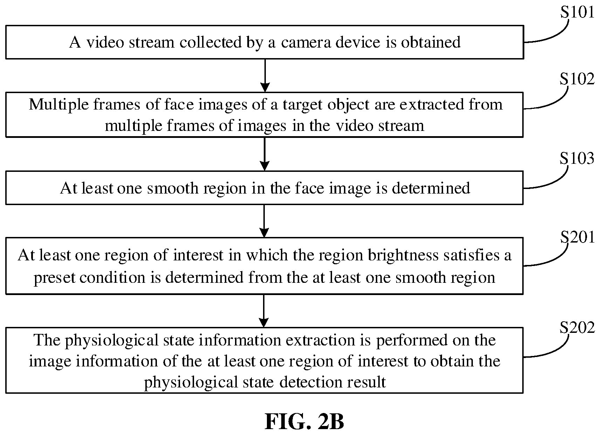

In some embodiments of the present application, in case that the region attribute information includes region brightness, the to-be-extracted information associated with the physiological state includes image information of at least one region of interest. In such case, the region filtering may be performed on the at least one smooth region based on the region brightness, so as to obtain the at least one region of interest. Then, the physiological state information extraction may be performed on the image information of the at least one region of interest, to obtain the physiological state detection result. FIG. 2B is a schematic flowchart of a second physiological state detection method provided by an embodiment of the present application. As shown in FIG. 2B, the operation S104 provided by the embodiment of the present application may be implemented by the following operation S201.

In operation S201, at least one region of interest in which the region brightness satisfies a preset condition is determined from the at least one smooth region.

The operation S105 provided by the aforementioned embodiment may be implemented by the following operation S202.

In operation S202, the physiological state information extraction is performed on the image information of the at least one region of interest to obtain the physiological state detection result.

In some embodiments of the present application, the operation that the filtering associated with the region of interest is implemented based on the image brightness threshold may be implemented by the following operations.

In a first operation, an image brightness threshold corresponding to the face image and region image brightness of each smooth region are determined.

In a second operation, the at least one smooth region is filtered according to the image brightness threshold and the region image brightness to obtain at least one candidate smooth region.

In a third operation, the at least one region of interest in which the region brightness satisfies a preset brightness condition is selected from the at least one candidate smooth region.

Firstly, the smooth regions may be filtered based on the image brightness threshold, and then the region of interest with the optimal brightness may be selected based on the candidate smooth regions obtained by filtering.

In order to implement effective filtering, in the embodiment of the present application, limitation may be performed by the image brightness threshold of the maximum brightness, and also may be performed by the image brightness threshold of the minimum brightness. With the former, smooth regions in which the region image brightness exceeds a first preset percentage of the maximum brightness may be filtered out. For example, smooth regions in which the region image brightness exceeds 90% of the maximum brightness may be filtered out, so as to filter out related smooth regions that may be overexposed. With the latter, smooth regions in which the region image brightness does not reach a second preset percentage of the minimum brightness may be filtered out. For example, smooth regions in which the region image brightness is less than 30% of the minimum brightness may be filtered out, so as to filter out related smooth regions that may be too dark.

The region image brightness involved above is a value. The value may refer to the average value of the region brightness, i.e., the value obtained by adding the pixel brightness values of all the pixels in the region and averaging. Alternatively, the value may also refer to the maximum value of the pixel brightness among all the pixels in the region. Alternatively, the value may also refer to the minimum value of the pixel brightness among all the pixels in the region. Alternatively, the value may also be a value obtained by calculating the pixel brightness values of all the pixels in the region according to a preset calculation manner.

In some embodiments of the present application, the maximum brightness and the minimum brightness may be preset brightness values, and different brightness values may be set for different application scenarios and/or different image formats, which may be adjusted according to requirements in a practical application. It is taken into account that within a certain brightness range, the higher the brightness is, the higher the possibility that the region has clearer image features is. Therefore, the obtained physiological detection result will be more accurate. Based on this, in the embodiment of the present application, the candidate smooth region with the maximum brightness may be selected as the smooth region.

In the embodiment of the present application, in case that multiple smooth regions are determined, firstly, the brightness weight of each smooth region may be determined, and then, the physiological state information extraction results of the multiple smooth regions may be fused based on the brightness weights. The related brightness weight may be determined based on the region image brightness of the corresponding region. For example, a smooth region with greater region image brightness may correspond to a greater brightness weight, and conversely, a smooth region with less region image brightness may correspond to a smaller brightness weight, such that the influence of the smooth region with greater brightness on the physiological state detection may be highlighted, and the influence of the smooth region with less brightness on the physiological state detection may be weakened. In this way, the physiological state detection result obtained by fusing will be more accurate.

In some embodiments of the present application, it is taken into account that different smooth regions may be affected by different external factors (e.g., illumination, or the like), and thus the respective reflected image features influenced by the physiological state are different from each other. In order to capture more effective face features as far as possible to implement more accurate physiological state detection, in case that the at least one region of interest in which the region brightness satisfies the preset condition is determined based on the at least one candidate smooth region, the physiological state information extraction may be performed based on the image information of the region of interest. The image information of the region of interest includes pixel brightness information of three different color channels. Then, the physiological state information extraction is performed on the image information of the at least one region of interest, so as to obtain the physiological state detection result of the target object. That is, the aforementioned operation S202 may be implemented by the following operations.

In a first operation, a time-domain signal representing the physiological state of the target object is determined based on the pixel brightness information of the three color channels of the region of interest.

In a second operation, frequency-domain conversion is performed on the time-domain signal to obtain a frequency-domain signal representing the physiological state of the target object.

In a third operation, a physiological state value of the target object is determined based on the peak value of the frequency-domain signal,.

In some embodiments of the present application, it is taken into account that the physiological state directly affects the blood flow change of the target object, and the blood flow change affects the brightness change of the image. Therefore, firstly, a time-domain brightness signal of the region of interest, which corresponds to each of three color channels, i.e., red, green and blue channels, may be determined, and an RGB three-dimensional signal may be formed. Then, a principal component analysis is performed on the time-domain brightness signals of the three different color channels, and the one-dimensional signal obtained after extracting the principal component (i.e., after dimension reduction) is taken as the time-domain signal representing the physiological state of the target object. The time-domain signal may be determined by the time-domain brightness signal of one of the aforementioned color channels (e.g., the green channel), and the selected channel may be the channel that best represents the blood flow change. That is, the operation that the time-domain signal representing the physiological state of the target object is determined based on the pixel brightness information of the three color channels of the region of interest in the aforementioned first operation may be implemented by the following process.

Firstly, a time-domain brightness signal, which corresponds to each color channel, of the smooth region is determined based on the pixel brightness information of the three color channels corresponding to the region of interest.

Secondly, principal component analysis is performed on the time-domain brightness signals, which correspond to the three different color channels, of the region of interest, to obtain the time-domain signal.

In some embodiments, in the process of determining the time-domain brightness signal of each region of interest corresponding to each color channel, image change information corresponding to multiple frames of face images in the video stream lasting for a period is required. It is because regarding physiological state information such as the heart rate, the respiratory rate, the blood oxygen, the blood pressure, or the like, a certain period for detection is often required, and that in the process of determining the time-domain brightness signal corresponding to the region of interest, the brightness values of multiple color channels may be used to improve the accuracy of the extraction of the time-domain brightness signal.

In order to implement a more accurate principal component analysis, processing, such as regularization, Detrend filter denoising, or the like, may be performed before the principal component analysis is performed on the three-dimensional time-domain brightness signal. In addition, after the principal component analysis, sliding average filter denoising processing may further be performed on the obtained time-domain signal, which may thereby improve the accuracy of the time-domain signal and improve the accuracy of the physiological state detection result obtained in the subsequent processing.

In some embodiments of the present application, it is taken into account that there is a difference in the pixel areas occupied by the generated images of different smooth regions due to the intersection angle between the face and the optical axis of the camera. Therefore, a semaphore closer to the actual situation may be obtained by the selected at least one smooth region and by weighting the pixel areas of the smooth region in the picture, and the detection accuracy may be improved.

In case that the smooth region of the face image is determined, with the physiological state detection method provided by the embodiment of the present application, firstly, the time-domain brightness signal corresponding to each smooth region may be determined, and then, each time-domain brightness signal may be weighted based on the area of the smooth region, thereby facilitating performing the physiological state information extraction based on the area-weighted time-domain brightness signal. The extracted physiological state detection result may be a detection result including at least one of the heart rate, the respiratory rate, the blood oxygen, the blood pressure, or the like. In addition, in case that the corresponding time-domain brightness signal for each smooth region is determined, the determined time-domain brightness signal may be weighted based on the area of the smooth region. The area-weighted time-domain brightness signal may highlight, to a greater extent, the influence of larger smooth regions in the face image on the physiological state detection, and may weaken the influence of smaller smooth regions in the face image on the physiological state detection, thereby improving the detection accuracy. In addition, the whole detection process does not require any specialized device, and the measurement may be performed anytime and anywhere, which has a better practicality.

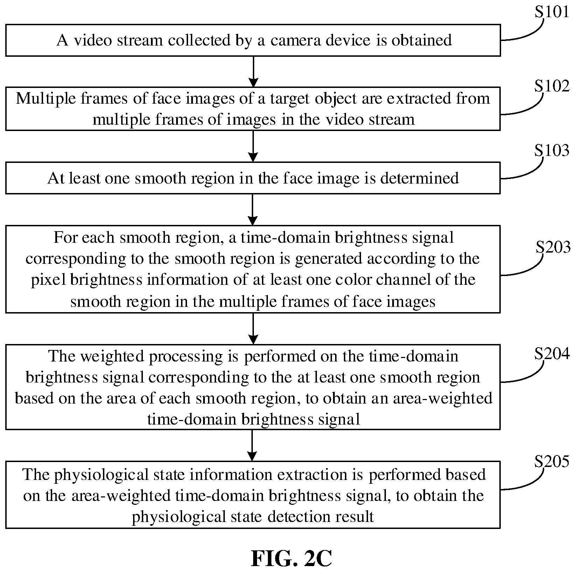

In some embodiments of the present application, in case that the region attribute information includes the region area, the to-be-extracted information associated with the physiological state may include a weighted time-domain brightness signal. In such case, the weighted processing may be performed on the time-domain brightness signal corresponding to the at least one smooth region based on the region area, so as to obtain the area-weighted time-domain brightness signal. Then, the physiological state information extraction may be performed based on the area-weighted time-domain brightness signal, so as to obtain the physiological state detection result. FIG. 2C is a schematic flowchart of a third physiological state detection method provided by an embodiment of the present application. As shown in FIG. 2C, the operation S104 provided by the embodiment of the present application may be implemented by the following operations S203 and S204.

In operation S203, for each smooth region, a time-domain brightness signal corresponding to the smooth region is generated according to the pixel brightness information of at least one color channel of the smooth region in the multiple frames of face images.

In operation S204, the weighted processing is performed on the time-domain brightness signal corresponding to the at least one smooth region based on the area of each smooth region, to obtain an area-weighted time-domain brightness signal.

The operation S105 provided by the aforementioned embodiment may be implemented by the following operation S205.

In operation S205, the physiological state information extraction is performed based on the area-weighted time-domain brightness signal, to obtain the physiological state detection result.

In some embodiments of the present application, in case that each smooth region having a relatively strong signal expression capability is determined, for each smooth region, the time-domain brightness signal of the smooth region corresponding to each color channel may be determined based on the brightness values of the three color channels corresponding to the smooth region in the multiple frames of face images. In this way, for each color channel, the weighted processing is performed on the time-domain brightness signal of the at least one smooth region in the color channel based on the area of each smooth region, so as to obtain the area-weighted time-domain brightness signal in the color channel. That is, the embodiment of the present application implements area-weighting in each color channel, such that each color channel has a relatively strong blood flow change expression capability. That is, the operation S203 provided by the embodiment of the present application may be implemented by the following process.

A time-domain brightness signal, which corresponds to each color channel, of the smooth region is determined based on the pixel brightness information, which corresponds to the three color channels, of the smooth region in the multiple frames of face images.

Correspondingly, the operation S204 provided by the aforementioned embodiment may be implemented by the following process.

For each color channel, the weighted processing is performed on the time-domain brightness signal of each of the at least one smooth region in the color channel based on the area of the smooth region, to obtain the area-weighted time-domain brightness signal in the color channel.

Furthermore, the operation S205 provided by the aforementioned embodiment may be implemented by the following process.

In a first operation, the principal component analysis is performed on the area-weighted time-domain brightness signal of the smooth region in multiple different color channels to obtain the time-domain signal representing the physiological state of the target object.

In a second operation, frequency-domain processing is performed on the time-domain signal representing the physiological state of the target object, and the physiological state detection result is determined based on the peak value of a frequency-domain signal obtained by the frequency-domain processing.

In some embodiments, it is taken into account that different region areas imply that the amount of information of the physiological features included in the corresponding smooth region is different. Therefore, the area weight of each smooth region may be determined based on the area of the corresponding smooth region, and the time-domain brightness signal corresponding to the at least one smooth region may be weighted by the area weight, so as to obtain a semaphore closer to the actual situation, thereby improving the accuracy of the generated physiological state detection result.

In the embodiment of the present application, for different face poses, the areas of different face sub-regions presented in the image are different, and the areas of the corresponding smooth regions are also different. Still taking the face tilted 45° to the left as an example, in such case, the left cheek region is almost invisible in the image, and the area of the corresponding smooth region is 0.

In some embodiments of the present application, the area of each smooth region may further be estimated according to the face pose and the parameters of the camera device. For example, after the detection of the face pose is completed by a neural network or the like, the face model facing the camera is projected into an image coordinate system according to parameters, such as the face pose angle, the focal length of the camera, or the like. Coordinates of boundary points in the image coordinate system, of each smooth region located based on the face key points, are calculated, and then the area value of each smooth region in the image is estimated. The area value may be used to estimate the area of each smooth region in the image in case that some regions of the face do not have enough brightness, or some key points of the face are missing. In the embodiment of the present application, the area of the smooth region may be determined based on the positions of the preset key feature points corresponding to the smooth region. In a practical application, the position of each preset key feature point may be taken as the vertex position of the smooth region. The length and width of the smooth region may be determined by the vertex distance, and then the region area may be determined. In this way, the determined region area is more accurate.

To some extent, a smooth region having a larger region area may carry more amount of information and may be assigned with a larger area weight. Conversely, to some extent, a smooth region having a smaller region area may carry less amount of information and may be assigned with a smaller area weight. In the embodiment of the present application, the area weight corresponding to each smooth region may be determined based on the proportion of each region area in the total region area, and then the area weighting may be performed.

Before the area weighting is performed, in order to ensure the extraction of the amount of effective information as far as possible, a smooth region having an area smaller than the threshold is removed in case that the at least one smooth region is determined. The threshold may be a preset fixed value, or may be a value determined according to the area distribution of the smooth regions in the entire face region in the current image. For example, a certain percentage (e.g., 20%) of the maximum value in the areas of the smooth regions in the current image may be taken as the threshold.

In a practical application, according to the face pose, a smooth region in which the visible range in the image does not satisfy a preset visibility requirement may be removed from the smooth regions. Still taking the face tilted 45° to the left as an example, in such case, the area of the right cheek region is smaller than the preset threshold and is almost invisible in the image, and thus the corresponding smooth region may be directly removed.

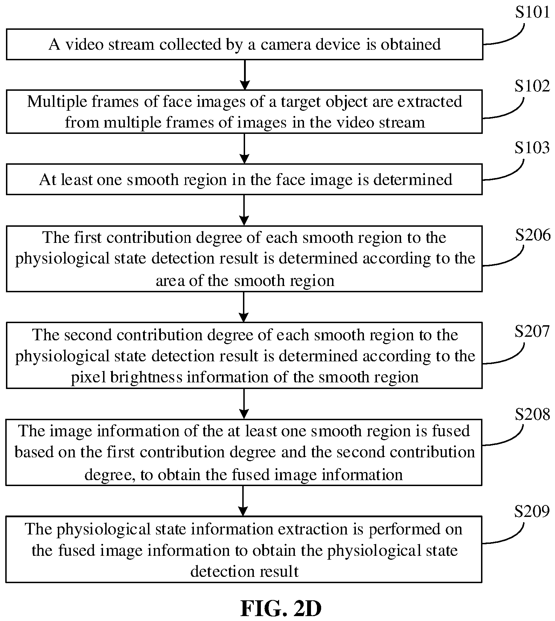

In case that the at least one smooth region of the face image is determined, in the physiological state detection method provided by the embodiment of the present application, in case that the region attribute information includes the region area and the region brightness, the to-be-extracted information associated with the physiological state includes fused image information. The fused image information indicates image information that fuses image factors on multiple dimensions. In such case, firstly, a first contribution degree of each smooth region to the physiological state detection result and a second contribution degree of each smooth region to the physiological state detection result may be determined. The first contribution degree indicates a contribution degree of the image information associated with the region area in the smooth region to the physiological state detection result, and the second contribution degree indicates the contribution degree of the image information associated with the region brightness in the smooth region to the physiological state detection result. Secondly, the image information of the at least one smooth region is fused based on the first contribution degree and the second contribution degree, and the fused image information is obtained. Thirdly, the physiological state information extraction is performed based on the fused image information. The extracted physiological state detection result may be a detection result including at least one of the heart rate, the respiratory rate, the blood oxygen, the blood pressure, or the like. In this way, the first contribution degree and the second contribution degree of each smooth region to the physiological state detection result may be determined respectively according to the area and the pixel brightness information of the smooth region, the image information of the at least one smooth region may be fused, and the physiological state information extraction may be performed based on the image information obtained by fusing, to obtain the physiological state detection result. In this way, in the process of performing the physiological state detection, the fusing of the image information of each smooth region may be implemented in combination with the region area and the region brightness, thereby improving the detection accuracy. FIG. 2D is a schematic flowchart of a fourth physiological state detection method provided by an embodiment of the present application. As shown in FIG. 2D, the operation S104 provided by the aforementioned embodiment may be implemented by the following operations S206 to S208.

In operation S206, the first contribution degree of each smooth region to the physiological state detection result is determined according to the area of the smooth region.

In operation S207, the second contribution degree of each smooth region to the physiological state detection result is determined according to the pixel brightness information of the smooth region.

In operation S208, the image information of the at least one smooth region is fused based on the first contribution degree and the second contribution degree, to obtain the fused image information.

The operation S105 provided by the aforementioned embodiment may be implemented by the following operation S209.

In operation S209, the physiological state information extraction is performed on the fused image information to obtain the physiological state detection result.

The related fusing process may be weighted fusing based on the first contribution degree and the second contribution degree. That is, for a smooth region, the higher the corresponding first contribution degree and the second contribution degree are, the stronger the representation capability of the fused image information corresponding to the smooth region is.

Alternatively, the aforementioned fusing process may further be performed based on a comparison result between the first contribution degree and the second contribution degree. In some embodiments, if the first contribution degree of a smooth region is much larger than the second contribution degree, the physiological state information extraction result corresponding to the smooth region may be selected to be determined based on the brightness information of the smooth region, and the influence of the area is ignored. If the first contribution degree of a smooth region is much smaller than the second contribution degree, the physiological state information extraction result corresponding to the smooth region may be determined only based on the area of the smooth region, and the influence of the brightness is ignored.

The related first contribution degree may be determined based on the area of the corresponding smooth region, and the value of the first contribution degree is proportional to the area of the corresponding smooth region. That is, to some extent, a smooth region having a larger area will provide a higher first contribution degree to the physiological state detection, and conversely, a smooth region having a smaller area will provide a lower first contribution degree to the physiological state detection. It is because as the region area increases, the amount of effective information that may be extracted will also increase, and to some extent, the increase in the amount of effective information may improve the accuracy of the physiological state detection.

Furthermore, the related second contribution degree may be determined based on the pixel brightness information of the corresponding smooth region, and the second contribution degree is proportional to the average pixel brightness value of the corresponding smooth region. That is, to some extent, a smooth region having a stronger pixel brightness will provide a higher second contribution degree to the physiological state detection, and conversely, a smooth region having a weaker pixel brightness will provide a lower second contribution degree to the physiological state detection. It is because as the pixel brightness increases, the corresponding image quality will also increase, and to some extent, a better image quality may also improve the accuracy of the physiological state detection. In addition, the second contribution degree is inversely proportional to the variance of the pixel brightness value of the corresponding smooth region. In such case, the corresponding second contribution degree may be determined based on the variance value.

In some embodiments, when the second contribution degree is determined based on the pixel brightness information, it is necessary to take into account potential extreme cases, such as overexposure or overdarkness. In such extreme cases, the accuracy of the physiological state detection may be affected. Based on this, in a practical application, firstly, a smooth region that is overexposed or too dark may be filtered out based on the pixel brightness, and then the second contribution degree may be determined for smooth regions that satisfy the brightness requirements, so as to ensure the accuracy of the final physiological state detection result.

In some embodiments, in case that the smooth regions are extracted from each frame of face image, on one hand, the first contribution degree corresponding to each smooth region may be determined based on the area of the smooth region, and on the other hand, the second contribution degree corresponding to each smooth region may be determined based on the pixel brightness information of the smooth region.

In case that the area of a smooth region is smaller than the preset area threshold, the first contribution degree of the smooth region is determined as 0. That is, a relatively small smooth region has a limited image representation capability, and the corresponding first contribution degree may be disregarded. Furthermore, in case that the average brightness value of each smooth region is determined according to the pixel brightness information of the smooth region, in case that the average brightness value is less than a first preset brightness threshold, the second contribution degree of the smooth region is determined as 0. Alternatively, in case that the average brightness value is greater than a second preset brightness threshold, the second contribution degree of the smooth region is determined as 0. That is, for a smooth region that may be overexposed or too dark, the corresponding second contribution degree may be disregarded.

Based on this, in case that the area of a smooth region belongs to a normal area and the smooth region is at a normal brightness, the corresponding area weight and brightness weight may be determined respectively. The area weight may be implemented according to the following operations.

In a first operation, an area sum value is obtained by summing the area of each smooth region.

In a second operation, for each smooth region, the ratio of the area of the smooth region to the area sum value is calculated, and an area proportion of the smooth region is obtained as the area weight corresponding to the smooth region.

The area sum value may be obtained after the area of each smooth region is summed. For a smooth region in which the area of the smooth region has a larger proportion in the area sum value, a larger area weight may be determined. Conversely, for a smooth region in which the area of the smooth region has a smaller proportion in the area sum value, a smaller area weight may be determined.

In addition, the related brightness weight may be implemented according to the following operations.

In a first operation, a brightness sum value is obtained by summing the average brightness value of each smooth region.

In a second operation, for each smooth region, the ratio of the average brightness value of the smooth region to the brightness sum value is calculated, and a brightness proportion of the smooth region is obtained as the brightness weight corresponding to the smooth region.

The brightness sum value may be obtained after the average brightness value of each smooth region is summed. For a smooth region in which the average brightness value of the smooth region has a larger proportion in the brightness sum value, a larger brightness weight may be determined. Conversely, for a smooth region in which the average brightness value of the smooth region has a smaller proportion in the brightness sum value, a smaller brightness weight may be determined.

In some embodiments of the present application, the image information of each smooth region may be fused based on the area weight and the brightness weight, so as to perform the subsequent physiological state detection. The first contribution degree includes the area weight, and the second contribution degree includes the brightness weight. The related fusing process, i.e., the aforementioned operation S208, may be implemented by the following process.

In a first operation, a respective total weight value corresponding to each smooth region is determined based on the product of the area weight and the brightness weight corresponding to the smooth region.

In a second operation, weighted fusing is performed on the image information of the at least one smooth region based on the corresponding total weight value, to determine the fused image information.

Firstly, a total weight value corresponding to each smooth region may be determined, and then, in case that the corresponding total weight value is assigned to the image information of each smooth region, the fused image information may be determined by a weighted summing.

In some embodiments of the present application, for a smooth region with a relatively large area weight and a relatively large brightness weight, the corresponding total weight value is larger, which, to some extent, may provide more information support for the fused image information. For a smooth region with a relatively large area weight and a relatively small brightness weight, or a smooth region with a relatively small area weight and a relatively large brightness weight, the corresponding total weight value may be larger or smaller, which is required to be determined based on the image analysis result. The fused image information obtained by the weighted fusing may furthest dig out effective pixel points on the face, which provides more data support for the subsequent physiological state detection, and thereby is conducive to improving the detection accuracy.

In some embodiments of the present application, in case that the first contribution degree and the second contribution degree of each smooth region to the physiological state detection result are determined, and the fused image information includes the fused time-domain brightness signal, with the physiological state detection method provided by the embodiment of the present application, firstly, the time-domain brightness signal corresponding to each smooth region may be determined by time-domain processing, and then, each time-domain brightness signal may be fused based on the first contribution degree and the second contribution degree of the smooth region, thereby facilitating performing the physiological state information extraction based on the fused time-domain brightness signal.

In some embodiments, in order to improve the accuracy of the physiological state detection, frequency-domain processing may be performed on the fused time-domain brightness signal. More useful information may be analyzed based on the frequency-domain signal obtained by the frequency-domain processing. For example, the amplitude distribution and energy distribution of each frequency component may be determined, and thereby frequency values of key amplitudes and the energy distribution may be obtained. A physiological state value of the target object may be determined based on the peak value of the frequency-domain signal.

In the embodiment of the present application, in case that the fused image information is determined, the time-domain brightness signal corresponding to each color channel may be determined based on the brightness values of the fused image information corresponding to the three color channels. After the principal component analysis is performed on each color channel, the physiological state detection associated with the target object may be implemented. That is, the aforementioned operation S208 may be further implemented by the following operations.

In a first operation, for each smooth region, time-domain processing is performed on the brightness value of the smooth region in the multiple frames of face images, to determine a time-domain brightness signal corresponding to the smooth region.

In a second operation, the time-domain brightness signals corresponding to multiple smooth regions are fused based on the first contribution degree and the second contribution degree, to obtain the fused time-domain brightness signal.

The operation S209 provided by the aforementioned embodiment may be implemented by the following process.

Frequency-domain processing is performed on the fused time-domain brightness signal, and the physiological state result is determined based on the peak value of a frequency-domain signal obtained by the frequency-domain processing.

In some embodiments, the operation that the frequency-domain processing is performed on the fused time-domain brightness signal may refer to the following operations. The principal component analysis is performed on the fused time-domain brightness signal to obtain a corresponding time-domain signal. Frequency-domain conversion is performed on the time-domain signal to obtain a frequency-domain signal representing the physiological state of the target object. The physiological state result of the target object is determined based on the peak value of the frequency-domain signal.

In some embodiments of the present application, in case that the video stream is obtained, firstly, the face image of the target object may be extracted from the video stream, and then the body archive data of the target object may be searched based on the face image. Furthermore, the time-domain processing and frequency-domain processing may be performed based on the image information of the face image, so as to obtain an estimated heart rate value of the target object, and the blood pressure value of the target object may be estimated based on the found body archive data and the processed estimated heart rate value, so as to obtain a blood pressure measurement result. In this way, compared with the related art in which there is a problem of measurement inconvenience as specialized instruments or devices are required, with the physiological state detection method provided by the embodiment of the present application, the estimated heart rate value for representing the fluctuation of the blood pressure may be determined, and then an accurate measurement associated with the blood pressure value may be implemented in combination with the body archive data. The measurement result is more accurate, and the measurement may be performed anytime and anywhere in real time without specialized devices, which has a better practicality. The Blood Pressure (BP) is the lateral pressure per unit area of the blood vessel wall when the blood is flowing in the blood vessel, which is a driving force pushing the blood to flow in the blood vessel, and is a very important physiological index reflecting the health condition of the human body in daily life.

In a practical application, it is taken into account that the heart pumps blood with each beat, the blood flows throughout the body along the aorta, and the pumped blood is the measured blood pressure. Generally, the level of the blood pressure is associated with the power of the heart pumping blood, that is, there is a certain association between the heartbeat condition and the blood pressure of the human body. Based on this, the image change information corresponding to multiple frames of face images in the video stream lasting for a period may be adopted to determine the estimated heart rate value, and then the blood pressure measurement result may be determined in combination with the body archive data. In this way, the measured blood pressure may be combined with the heartbeat condition and specific attributes of different human bodies, thereby better matching requirements of actual scenarios.

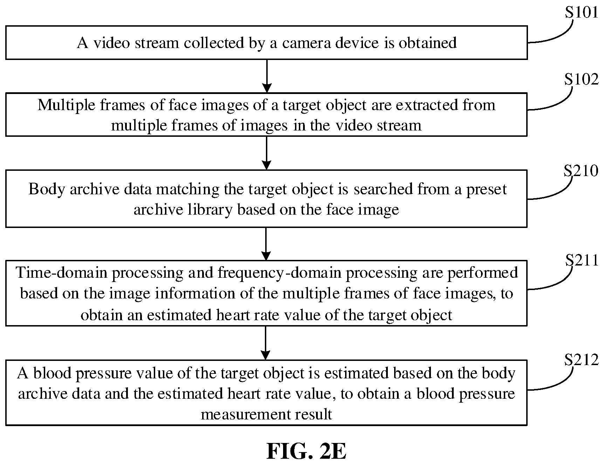

In some embodiments of the present application, in case that the physiological state detection result of the target image includes the blood pressure measurement result, the aforementioned embodiment may perform the following operations S210 to S212. FIG. 2E is a schematic flowchart of a fifth physiological state detection method provided by an embodiment of the present application.

In operation S210, body archive data matching the target object is searched from a preset archive library based on the face image.

In operation S211, time-domain processing and frequency-domain processing are performed based on the image information of the multiple frames of face images, to obtain an estimated heart rate value of the target object.

In operation S212, a blood pressure value of the target object is estimated based on the body archive data and the estimated heart rate value, to obtain a blood pressure measurement result.

In the embodiment of the present application, the related body archive data may include data such as the height, the body weight, the gender data, the age, or the like. It is because human bodies with different heights, body weights, gender data, and ages have different physical function conditions. For example, the heartbeat of a child is generally faster than that of an elderly person. The operation that the blood pressure value is estimated in combination with the body archive data and the estimated heart rate value is intended to implement a blood pressure measurement that is adaptable to various types of human bodies and has a better adaptability.

In some embodiments of the present application, the aforementioned operation S210 may be implemented by at least one of the following manners.

In a first manner, based on face features of the target object extracted from the face image, body archive data corresponding to the face features of the target object is searched from the preset archive library.

In a second manner, based on an identifier of the target object identified from the face image, body archive data corresponding to the identifier is searched from the preset archive library.

In the embodiment of the present application, the body archive data matching the target object may be searched from the preset archive library based on the face image, that is, the body archive data of each object may be stored in the preset archive library in advance. Once the face image is obtained, the body archive data corresponding to the target object may be searched from the preset archive library based on the face image or the face features extracted from the face image, which is easy to operate. Alternatively, some body archive data, such as the age, the gender, or the like, may further be obtained by analyzing the face image of the target object.

In some embodiments of the present application, the face features of the target object may be extracted based on the face image, and then the body archive data corresponding to the face features of the target object may be searched from the preset archive library. The related face features may be related features including the eye distance, the eyebrow and eye distance, or the like, or may be feature representations obtained by performing feature extraction on the face image through a deep learning model for face recognition, such as a convolutional neural network. In case that the face features of each object are recorded in the preset archive library in advance, the body archive data corresponding to the target object may be searched out based on the similarity between the extracted face features and each face feature in the preset archive library.

In some embodiments of the present application, the target object may be identified based on the face image to determine the identifier of the target object, and then the body archive data corresponding to the identifier may be searched from the preset archive library as the body archive data matching the target object. That is, different identifier may be set for each object in advance. In case that face comparison is performed based on the face image, the target object corresponding to the identifier, and its body archive data may be determined.

In some embodiments of the present application, the related estimated heart rate value may be obtained by performing the time-domain processing and frequency-domain processing based on the image information of the extracted multiple frames of face images. In a practical application, an estimated heart rate value that better satisfies actual requirements may be obtained based on the time-domain processing and frequency-domain processing of the image information of multiple color channels. The implementation process may be referred to the process that the physiological state information extraction is performed based on the image information value of the smooth region to obtain the physiological state detection result of the target object. The physiological state detection result is the estimated heart rate value.

In some embodiments of the present application, the process of implementing the blood pressure measurement result of the target object, i.e., the aforementioned operation S212, may be implemented by the following operations.

In a first operation, an ejection time corresponding to the estimated heart rate value is determined according to a linear fitting relationship between the ejection time and a heart rate. A body surface area corresponding to the target object is calculated based on gender data, a height and a body weight included in the body archive data. A heart elasticity value of the target object is determined based on the body weight and an age included in the body archive data and the estimated heart rate value of the target object.

In a second operation, a stroke volume corresponding to the target object is determined based on the ejection time, the body surface area, the age included in the body archive data, and the estimated heart rate value.

In a third operation, an estimated pulse pressure value corresponding to the target object is calculated based on a ratio between the stroke volume corresponding to the target object and the heart elasticity value.

In a fourth operation, a systolic pressure value and a diastolic pressure value of the target object are calculated based on the estimated pulse pressure value corresponding to the target object and the gender data included in the body archive data.

In some embodiments, the measured blood pressure including a systolic pressure value and a diastolic pressure value may be implemented in combination with each body archive data of the object and the estimated heart rate value. In order to facilitate the understanding of the aforementioned process of the blood pressure measurement, the following is the related terminology in the blood pressure measurement result.

Systolic Pressure: when the heart contracts to pump blood from the ventricle into the artery, the pressure generated by the blood flow against the arterial wall is the systolic pressure.

Diastolic Pressure: when the heart is diastolic, the arterial blood vessel wall has a certain elasticity, and the pressure when the blood flow is pushed to continuously flow forward is called the diastolic pressure.

Pulse Pressure: the difference between the systolic pressure and the diastolic pressure.

Mean Arterial Pressure: the average arterial blood pressure during a cardiac cycle.

Stroke Volume (SV): the amount of blood ejected by the ventricle on one side in a cardiac cycle.

Ejection Time: i.e., The Left Ventricular Ejection Time (LVET), defined as the time interval from the aortic valve opening to the aortic valve closure, and is the systolic period during which the left ventricle ejects blood into the aorta, which may be abbreviated as ET.

Body Surface Area (BSA): Stevenson's formula is a body surface area formula that fits characteristics of a human body.

Cardiac Output (CO): describes the amount of blood pumped by the heart per unit of time.

Systemic Vascular Resistance (SVR).

In some embodiments, illustration may be performed in combination with specific formulas. For example, the ejection time corresponding to the estimated heart rate value is determined according to the linear fitting relationship between the ejection time and the heart rate. The linear fitting relationship here is ET=364.5-1.23*HR. ET is used to represent the ejection time, HR is used to represent the estimated heart rate value, and the parameters such as 364.5 and 1.23 may be the result of performing a linear fitting according to a specific dataset test.

In some embodiments, the body surface area corresponding to the target object may be calculated according to the gender data, the height and the body weight included in the archive data. The BSA is calculated according to the health archive information (Height and Weight) of the target object and Stevenson's formula.

In some embodiments, the body surface area of a male target object may be determined according to BSA=0.0057*Height+0.0121*Weight+0.0882, and the body surface area of a female target object may be determined according to BSA=0.0073*Height+0.0127*Weight-0.2106.

In case that the ET, HR and BSA are determined, the SV corresponding to the target object may be determined in combination with the Age included in the body archive data. The SV may be determined according to the following formula (1).

$$SV = -6.6 + 0.25*(ET-35) - 0.62*HR + 40.4*BSA - 0.51*Age \qquad \text{formula (1)}$$

The estimated Pulse pressure (Pp) value corresponding to the target object may be calculated based on the ratio between the SV and the Cardiac Elasticity (CE) corresponding to the target. The pulse pressure may be determined by the following formula (2).

$$Pp = SV/CE \qquad \text{formula (2)}$$

Here, CE=0.013*Weight-0.007*Age-0.004*HR+1.307, and Pm is calculated according to the mean arterial pressure Pm=CO*SVR, where CO is the cardiac output and SVR is the systemic vascular resistance. The average of CO is 5 L/min for the male and 4.5 L/min for the female, and the empirical value of SVR is 18.31 mmHgmin/L.

In case that the mean arterial pressure is determined, the systolic pressure (Ps=Pm+2/3Pp) and the diastolic pressure (Pd=Pm-1/3Pp) may be calculated. Derivation of the two formulas are shown in formula (3).

$$Pm=Pd+1/3(Ps-Pd) \qquad \text{formula (3)}$$

The computing formulas of Ps and Pd may be derived in combination with the definition of Pp, i.e., Pp=Ps-Pd,.

In order to better monitor the blood pressure condition of the target object, the blood pressure measurement result and collection time corresponding to multiple frames of images may be stored into a blood pressure measurement record of the target object. The blood pressure measurement record may store the blood pressure measurement conditions of the target object during various periods. A corresponding blood pressure measurement report may further be generated based on the blood pressure measurement record within a certain preset duration, thereby facilitating monitoring the target object in real time. In particular, in the process of driving, phenomenon of dangerous driving behaviors, which is caused by a too high blood pressure or a too low blood pressure, may be reduced by monitoring the blood pressure in real time, and thus driving safety is improved.

The related blood pressure measurement report may be, for example, generated by smoothing multiple measurement results in 5 minutes, or a generated dynamic change report of the blood pressure during a period (e.g., comparison and trend of change of the blood pressure during multiple different periods in one day). In addition, the blood pressure measurement report may further be a report of monitoring the blood pressure on other dimensions, which is not specifically limited herein.

In a practical application, the related blood pressure measurement result may be stored in association with the body archive data of the target object. As various body archive data, such as the age and the body weight, changes, the blood pressure measurement result will also change, which facilitates performing the body condition analysis of a longer time span on the target object, and enables a timely alert in case of an abnormal body index.

After the blood pressure measurement is implemented, with the embodiment of the present application, the blood pressure measurement result may further be displayed, such that better vehicle cabin service is provided to the target object by the displayed blood pressure measurement result.

In some embodiments, in order to improve the accuracy of the physiological state detection, the frequency-domain conversion may be performed on the time-domain signal. More useful information may be analyzed based on the converted frequency-domain signal. For example, the amplitude distribution and energy distribution of each frequency component may be determined, and thereby frequency values of key amplitudes and the energy distribution may be obtained. The physiological state value of the target object may be determined based on the peak value of the frequency-domain signal.

Taking the heart rate detection as an example, the peak value of the frequency-domain signal (pmax) may be determined, and the measured raw heart rate may be obtained by the result of summing pmax and a heart rate reference value. pmax represents variation of the heart rate, and the heart rate reference value may be determined by the lower limit of an experiential estimated heart rate range. Furthermore, influence of factors, such as the frame rate of the video, the length of the frequency-domain signal, or the like, may further be taken into account to adjust the heart rate reference value.

After the heart rate is determined, related physiological indexes, such as oxygen saturation and heart rate variability, may be measured. For the oxygen saturation, the time-domain signals of $HbO_2$ and Hb may be detected respectively by the red light region (600 to 800 nm) and near red light region (800 to 1000 nm), and then the corresponding ratio may be calculated to obtain the oxygen saturation. For the heart rate variability, after the time-domain signals are extracted, a number of intervals are obtained by calculating the spacing between each two adjacent peaks and combining with the frame rate, and then the standard deviation of these intervals (Standard Deviation of NN Intervals, SDNN) is taken. In this way, the heart rate variability is obtained.

The method of respiratory rate detection is similar to that of the heart rate detection. The difference is that the range of the respiratory rate is different from the range of the heart rate, and the corresponding reference values are set differently. The respiratory rate detection may be implemented based on the same method as described above.

With the embodiment of the present application, the physiological state detection for multiple frames of images is implemented, that is, the image change information corresponding to the multiple frames of images may represent the change of the physiological state. In a practical application, the physiological state detection result determined by the related video stream may be updated as collection of image frames continues.

In some embodiments of the present application, in case that one or more frames of images included in a new video stream are obtained, the face detection may be performed on the images in the new video stream. Face images of the target object in the vehicle cabin are extracted, and at least one smooth region in each frame of face image is determined. Weighted processing or region filtering is performed on the at least one smooth region based on region attribute information, to obtain to-be-extracted information associated with a physiological state. The physiological state detection result is updated based on the to-be-extracted information. If the preset detection duration is not reached, the update is performed again based on the obtained new video stream, until the preset detection duration is reached and an updated physiological state detection result is obtained.

In case that one or more frames of images included in a new video stream are obtained, the face detection may be performed on the images in the new video stream. Face images of the target object in the vehicle cabin are extracted, and at least one smooth region in the face image is determined. At least one region of interest in which the region brightness satisfies the preset condition is determined from the at least one smooth region. In this way, the physiological state detection result may be updated based on the image information of the at least one region of interest. If the preset detection duration is not reached, the update is performed again based on the obtained new video stream, until the preset detection duration is reached and the updated physiological state detection result is obtained.

In some embodiments, in case that one or more frames of images included in a new video stream are obtained, the face detection may be performed on the images in the new video stream. Face images of the target object in the vehicle cabin are extracted, and at least one smooth region in the face image is determined. For each smooth region, a time-domain brightness signal corresponding to the smooth region is generated according to the pixel brightness information of at least one color channel of the smooth region in multiple frames of face images. Weighted processing is performed on the time-domain brightness signal corresponding to the at least one smooth region based on the area of each smooth region, to obtain an area-weighted time-domain brightness signal, and the physiological state detection result may be updated based on the area-weighted time-domain brightness signal. If the preset detection duration is not reached, the update is performed again based on the obtained new video stream, until the preset detection duration is reached and the updated physiological state detection result is obtained.

In some embodiments, in case that one or more frames of images included in a new video stream are obtained, the face detection may be performed on the images in the new video stream. Face images of the target object in the vehicle cabin are extracted, and at least one smooth region in the face image is determined. In case that a first contribution degree and a second contribution degree of each smooth region to the physiological state detection result are determined, the image information of the at least one smooth region is fused based on the first contribution degree and the second contribution degree, to obtain fused image information. The physiological state detection result may be updated based on the fused image information. If the preset detection duration is not reached, the update is performed again based on the obtained new video stream, until the preset detection duration is reached and the updated physiological state detection result is obtained.

The heart rate detection is still illustrated as an example. In case that the preset detection duration is determined as 30s, the video stream may be obtained continuously during the 30s. In case that the measured heart rate is calculated based on multiple frames of images of the initial video stream (e.g., the video stream within the initial 5 seconds), it is still within 30 seconds. In such case, the number of image frames increases as image frames are collected, and a new measured heart rate may be calculated every time a frame is added or n frames are added. Smoothing processing is then performed through moving average. The measurement is ended when 30s is reached, and the final measurement result is obtained.

As an example, in the vehicle cabin environment, in order to help the target object perform a faster physiological state measurement, during a physiological state detection process, a detection process reminder signal for reminding the target object of the required detection duration may be generated based on the duration of the obtained video stream and the preset detection duration. For example, if the duration of the obtained video stream (i.e., the detection time that the physiological state detection of the current target object has lasted) reaches 25 seconds and the preset detection duration is 30 seconds, a voice or a ScreenTip of "please hold still, the detection will be completed in 5 seconds" may be issued. Alternatively, when the duration of the physiological state detection of the current target object reaches 30 seconds, a voice or a ScreenTip of "measurement completed" may be issued.

After the physiological state detection is implemented, with the embodiment of the present application, the physiological state detection result may further be displayed to provide a better vehicle cabin service to the target object through the displayed physiological state detection result.

In the embodiment of the present application, the physiological state detection result of the target object may be transmitted to a display screen in the vehicle cabin, to display the physiological state detection result on the display screen. In this way, a person in the vehicle cabin may monitor his own physiological state in real time, and may further seek medical advice or take other necessary measures timely in case of an abnormal physiological state. Furthermore, the physiological state detection result of the target object may be transmitted to a server side of a physiological state detection application, to send the physiological state detection result through the server side to a terminal device used by the target object in case that the target object requests to obtain the detection result through the physiological state detection application.

That is, the physiological state detection result of the target object may be recorded on the server, and statistical analysis may further be performed on the physiological state detection result on the server side. For example, the physiological state statistical result of a historical month or week may be determined. In this way, in case that the target object initiates a physiological state detection application request, the physiological state detection result, the statistical result, or the like may be sent to the terminal device of the target object, so as to implement a more comprehensive physiological state assessment.

The aforementioned physiological state detection application may be a specific Application (APP) for performing the physiological state detection. With the APP, an obtaining request of the detection result associated with the target object may be responded, and then result presentation on the APP may be implemented, which has a better practicality.

It is to be understood by those skilled in the art that in the aforementioned methods, the written order of the operations does not imply a strict execution order, and does not constitute any limitation on the implementation process. The specific execution order of the operations should be determined by the function and potential internal logic.

Based on the same inventive concept, a physiological state detection apparatus corresponding to the physiological state detection method is further provided by an embodiment of the present application. Since the apparatus in the embodiment of the present application solve problems by a principle similar to the aforementioned physiological state detection method in the embodiment of the present application, the implementations of the apparatus may be referred to the implementations of the method.

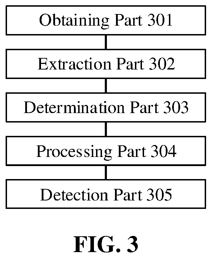

FIG. 3 is a schematic diagram of a physiological state detection apparatus provided by an embodiment of the present application. As shown in FIG. 3, the apparatus includes an obtaining part 301, an extraction part 302, a determination part 303, a processing part 304 and a detection part 305. The obtaining part 301 is configured to obtain a video stream collected by a camera device. The extraction part 302 is configured to extract multiple frames of face images of a target object from multiple frames of images in the video stream. The determination part 303 is configured to determine at least one smooth region in the face image. The processing part 304 is configured to perform weighted processing or region filtering on the at least one smooth region based on region attribute information, to obtain to-be-extracted information associated with a physiological state. The detection part 305 is configured to perform physiological state information extraction based on the to-be-extracted information to obtain a physiological state detection result of the target object.

In some embodiments of the present application, the determination part 303 is further configured to perform face feature point extraction on the face image to obtain positions of multiple preset key feature points in the face image; and determine, based on the positions of the multiple preset key feature points, at least one smooth region in the face image corresponding to the multiple preset key feature points.

In some embodiments of the present application, the region attribute information includes region brightness, and the to-be-extracted information associated with the physiological state includes image information of at least one region of interest. The processing part 304 is further configured to determine, from the at least one smooth region, at least one region of interest in which the region brightness satisfies a preset condition. The detection part 305 is further configured to perform the physiological state information extraction on the image information of the at least one region of interest to obtain the physiological state detection result.

In some embodiments of the present application, the processing part 304 is further configured to determine an image brightness threshold corresponding to the face image and region image brightness of each smooth region; filter the at least one smooth region according to the image brightness threshold and the region image brightness, to obtain at least one candidate smooth region; and select, from the at least one candidate smooth region, the at least one region of interest in which the region brightness satisfies a preset brightness condition.

In some embodiments of the present application, the image information of the region of interest includes pixel brightness information of three different color channels. The detection part 305 is further configured to determine, based on the pixel brightness information of the three color channels of the region of interest, a time-domain signal representing the physiological state of the target object; perform frequency-domain conversion on the time-domain signal to obtain a frequency-domain signal representing the physiological state of the target object; and determine, based on a peak value of the frequency-domain signal, the physiological state detection result of the target object.

In some embodiments of the present application, the detection part 305 is further configured to determine, based on the pixel brightness information of the three color channels corresponding to the region of interest, a time-domain brightness signal, which corresponds to each color channel, of the region of interest; and perform principal component analysis on the time-domain brightness signals, which correspond to the three different color channels, of the region of interest, to obtain the time-domain signal.

In some embodiments of the present application, the region attribute information includes a region area, and the to-be-extracted information associated with the physiological state includes a weighted time-domain brightness signal. The processing part 304 is further configured to generate, for each smooth region, a time-domain brightness signal corresponding to the smooth region, according to pixel brightness information of at least one color channel of the smooth region in the multiple frames of face images; and perform, based on an area of each smooth region, the weighted processing on the time-domain brightness signal corresponding to the at least one smooth region, to obtain an area-weighted time-domain brightness signal. The detection part 305 is further configured to perform, based on the area-weighted time-domain brightness signal, the physiological state information extraction, to obtain the physiological state detection result.

In some embodiments of the present application, the smooth region corresponds to pixel brightness information of three different color channels. The processing part 304 is further configured to determine, based on the pixel brightness information, which corresponds to the three color channels, of the smooth region in the multiple frames of face images, a time-domain brightness signal, which corresponds to each color channel, of the smooth region; and perform, for each color channel, the weighted processing on the time-domain brightness signal of each of the at least one smooth region in the color channel based on the area of the smooth region, to obtain the area-weighted time-domain brightness signal in the color channel. The detection part 305 is further configured to perform principal component analysis on the area-weighted time-domain brightness signal of the smooth region in multiple different color channels to obtain a time-domain signal representing the physiological state of the target object; and perform frequency-domain processing on the time-domain signal representing the physiological state of the target object, and determine the physiological state detection result based on a peak value of a frequency-domain signal obtained by the frequency-domain processing.

In some embodiments of the present application, the region attribute information includes an region area and region brightness, and the to-be-extracted information associated with the physiological state includes fused image information. The processing part 304 is further configured to determine, according to an area of each smooth region, a first contribution degree of the smooth region to the physiological state detection result; determine, according to pixel brightness information of each smooth region, a second contribution degree of the smooth region to the physiological state detection result; and fuse, based on the first contribution degree and the second contribution degree, image information of the at least one smooth region, to obtain the fused image information. The detection part 305 is further configured to perform the physiological state information extraction on the fused image information to obtain the physiological state detection result.

In some embodiments of the present application, the first contribution degree includes an area weight, and the second contribution degree includes a brightness weight. The processing part 304 is further configured to determine, based on a product of the area weight and the brightness weight corresponding to each smooth region, a total weight value corresponding to the smooth region; and perform, based on the corresponding total weight value, weighted fusing on the image information of the at least one smooth region, to determine the fused image information.

In some embodiments of the present application, the fused image information includes a fused time-domain brightness signal. The processing part 304 is further configured to perform, for each smooth region, time-domain processing on a brightness value of the smooth region in the multiple frames of face images, to determine a time-domain brightness signal corresponding to the smooth region; and fuse, based on the first contribution degree and the second contribution degree, the time-domain brightness signals corresponding to multiple smooth regions, to obtain the fused time-domain brightness signal. The detection part 305 is configured to perform frequency-domain processing on the fused time-domain brightness signal, and determine the physiological state result based on a peak value of a frequency-domain signal obtained by the frequency-domain processing.

In some embodiments of the present application, the processing part 304 is further configured to search, based on the face image, body archive data matching the target object from a preset archive library. The detection part 305 is further configured to perform, based on image information of the multiple frames of face images, time-domain processing and frequency-domain processing, to obtain an estimated heart rate value of the target object; and estimate, based on the body archive data and the estimated heart rate value, a blood pressure value of the target object, to obtain a blood pressure measurement result.

In some embodiments of the present application, the processing part 304 is further configured to search, based on face features of the target object extracted from the face image, body archive data corresponding to the face features of the target object from the preset archive library; and search, based on an identifier of the target object identified from the face image, body archive data corresponding to the identifier from the preset archive library.

In some embodiments of the present application, the detection part 305 is further configured to determine an ejection time corresponding to the estimated heart rate value according to a linear fitting relationship between the ejection time and a heart rate; calculate a body surface area corresponding to the target object based on gender data, a height and a body weight included in the body archive data; determine a heart elasticity value of the target object based on the body weight and an age included in the body archive data and the estimated heart rate value of the target object; determine a stroke volume corresponding to the target object based on the ejection time, the body surface area, the age included in the body archive data, and the estimated heart rate value; calculate an estimated pulse pressure value corresponding to the target object based on a ratio between the stroke volume corresponding to the target object and the heart elasticity value; and calculate a systolic pressure value and a diastolic pressure value of the target object based on the estimated pulse pressure value corresponding to the target object and the gender data included in the body archive data.

In some embodiments of the present application, the extraction part 302 is further configured to determine, according to a face detection result of the video stream and a designated riding position, the face image of the target object from detected face images. The designated riding position is used to indicate a position of the target object which is being measured.

In some embodiments of the present application, the apparatus further includes a display part and a sending part. The display part is configured to transmit the physiological state detection result of the target object to a display screen in the vehicle cabin, to display the physiological state detection result on the display screen. The sending part is configured to transmit the physiological state detection result of the target object to a server side of a physiological state measurement application, to send the physiological state detection result through the server side to a terminal device used by the target object in case that the target object requests to obtain the detection result through the physiological state application.

In some embodiments of the present application, the processing part 304 is further configured to perform the following operations repeatedly in case that a new video stream is obtained, until a preset detection duration is reached and an updated physiological state detection result is obtained. Multiple frames of face images of the target object are extracted from the new video stream. At least one smooth region in each frame of face image is determined. Weighted processing or region filtering is performed on the at least one smooth region based on region attribute information, to obtain to-be-extracted information associated with a physiological state. The physiological state detection result is updated based on the to-be-extracted information.

In some embodiments of the present application, the apparatus includes a reminder part. The reminder part is configured to generate a detection process reminder signal according to a duration of the obtained video stream and the preset detection duration. The detection process reminder signal is used to remind the target object of a required detection duration.

Descriptions regarding processing flows of various parts and interaction flows between various parts in the apparatus may be referred to the related illustrations in the aforementioned method embodiments, and will not be described in detail herein.

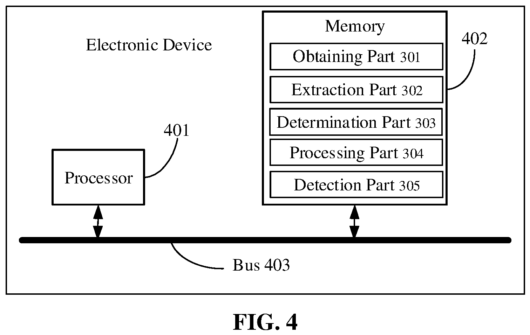

An embodiment of the present application further provides an electronic device. FIG. 4 is a schematic structure diagram of the electronic device provided by the embodiment of the present application. As shown in FIG. 4, the electronic device includes a processor 401, a memory 402 and a bus 403. The memory 402 has stored thereon machine-readable instructions executable by the processor 401 (e.g., execution instructions corresponding to the obtaining part 301, the extraction part 302, the determination part 303, the processing part 304 and the detection part 305 of the apparatus in FIG. 3). The processor 401 and the memory 402 communicate through the bus 403 when the electronic device is in operation, and the machine-readable instructions, when executed by the processor 401, perform the following processing.

A video stream collected by a camera device is obtained.

Multiple frames of face images of a target object are extracted from multiple frames of images in the video stream.

At least one smooth region in the face image is determined.

Weighted processing or region filtering is performed on the at least one smooth region based on region attribute information, to obtain to-be-extracted information associated with a physiological state.

Physiological state information extraction is performed based on the to-be-extracted information to obtain a physiological state detection result of the target object.

An embodiment of the present application further provides a computer-readable storage medium having stored thereon a computer program. The computer program, when run by a processor, performs the physiological state detection method described in the aforementioned method embodiments. The storage medium may be a volatile or non-volatile computer-readable storage medium.

An embodiment of the present application further provides a computer program product carrying program codes. Instructions included in the program codes may be used to perform the physiological state detection method described in the aforementioned method embodiments.

An embodiment of the present application further provides a computer program including computer-readable codes. In case that the computer-readable codes are run in an electronic device, a processor of the electronic device performs the physiological state detection method described in the aforementioned method embodiments.

The aforementioned computer program product may be specifically implemented by means of hardware, software, or a combination thereof. In an optional embodiment, the computer program product is specifically embodied as a computer storage medium. In another optional embodiment, the computer program product is specifically embodied as a software product, such as a Software Development Kit (SDK), or the like.

Those skilled in the art may clearly understand that for a convenient and brief description, specific operation processes of the systems and apparatuses described above may be referred to corresponding processes in the aforementioned method embodiments. In several embodiments provided by the present application, it is to be understood that the disclosed systems, apparatuses and methods may be implemented in other manners. The apparatus embodiments described above are only schematic. For example, division of the units is only a kind of logic function division, and other division manners may be adopted during a practical implementation. For example, multiple units or components may be combined or integrated into another system, or some features may be neglected or not executed. In addition, coupling or direct coupling or communication connection between various displayed or discussed components may be indirect coupling or communication connection through some communication interfaces, apparatuses or units, and may be electrical, mechanical, or in other forms.

Units described as separate components may or may not be physically separated, and components displayed as units may or may not be physical units, that is, may be located in the same place, or may also be distributed to multiple network units. Part or all of the units may be selected according to a practical requirement to implement the purpose of the solutions of the embodiments.

In addition, various functional units in various embodiments of the present application may be integrated into a processing unit, or each unit may physically exist independently, or two or more than two units may be integrated into a unit.

When implemented in form of a software functional unit and sold or used as an independent product, the functions may be stored in a non-volatile computer-readable storage medium that is executable by a processor. Based on such understanding, the technical solutions of the present application substantially or parts making contributions to the related art or part of the technical solutions may be embodied in form of a software product. The computer software product is stored in a storage medium, including several instructions for enabling an electronic device (which may be a personal computer, a server, a network device, or the like) to perform all or part of the operations of the methods in various embodiments of the present application. The abovementioned storage medium includes various media capable of storing program codes, such as a USB disk, a mobile hard disk, a Read-Only Memory (ROM), a Random Access Memory (RAM), a magnetic disk, an optical disk, or the like.

Finally, it is to be noted that the embodiments described above are only specific implementations of the present application and used to illustrate the technical solutions of the present application, but are not used to limit the present application. The scope of protection of the present application is not limited thereto. Although the present application is illustrated in detail with reference to the foregoing embodiments, it is to be understood by those of ordinary skill in the art that within the technical scope disclosed in the present application, it is still possible for those of ordinary skill in the art to make modifications or readily conceivable changes to the technical solutions recorded in the foregoing embodiments, or to make equivalent substitutions to some of the technical features. These modifications, changes or substitutions do not make the essence of the corresponding technical solutions depart from the spirit and scope of the technical solutions of the embodiments of the present application, and should all fall within the scope of protection of the present application. Therefore, the scope of protection of the present application should be subject to the scope of protection of the claims.

INDUSTRIAL APPLICABILITY

The embodiments of the present application provide a physiological state detection method and apparatus, an electronic device, a storage medium, and a program. The method includes the following operations. A video stream collected by a camera device is obtained. Multiple frames of face images of a target object are extracted from multiple frames of images in the video stream. At least one smooth region in the face image is determined. Weighted processing or region filtering is performed on the at least one smooth region based on region attribute information, to obtain to-be-extracted information associated with a physiological state. Physiological state information extraction is performed on the to-be-extracted information to obtain a physiological state detection result of the target object.

**Claims**

1.  A physiological state detection method, comprising:

    obtaining a video stream collected by a camera device;
    extracting a plurality of frames of face images of a target object from a plurality of frames of images in the video stream;
    determining at least one smooth region in the face image;
    performing, based on region attribute information, weighted processing or region filtering on the at least one smooth region, to obtain to-be-extracted information associated with a physiological state; and

performing physiological state information extraction on the to-be-extracted information to obtain a physiological state detection result of the target object.

2. The method of claim 1, wherein determining the at least one smooth region in the face image comprises:

   performing face feature point extraction on the face image to obtain positions of a plurality of preset key feature points in the face image; and
   determining, based on the positions of the plurality of preset key feature points, at least one smooth region in the face image corresponding to the plurality of preset key feature points.

3. The method of claim 1 or 2, wherein the region attribute information comprises region brightness, the to-be-extracted information comprises image information of at least one region of interest, and performing, based on the region attribute information, the weighted processing or the region filtering on the at least one smooth region comprises:

   determining, from the at least one smooth region, at least one region of interest in which the region brightness satisfies a preset condition;
   wherein performing the physiological state information extraction on the to-be-extracted information to obtain the physiological state detection result of the target object comprises:
   performing the physiological state information extraction on the image information of the at least one region of interest to obtain the physiological state detection result.

4. The method of claim 3, wherein determining, from the at least one smooth region, the at least one region of interest in which the region brightness satisfies the preset condition comprises:

   determining an image brightness threshold corresponding to the face image and region image brightness of each smooth region;
   filtering the at least one smooth region according to the image brightness threshold and the region image brightness, to obtain at least one candidate smooth region; and
   selecting, from the at least one candidate smooth region, the at least one region of interest in which the region brightness satisfies a preset brightness condition.

5. The method of claim 3 or 4, wherein the image information of the region of interest comprises pixel brightness information of three different color channels, and performing the physiological state information extraction on the image information of the at least one region of interest to obtain the physiological state detection result comprises:

   determining, based on the pixel brightness information of the three color channels of the region of interest, a time-domain signal representing the physiological state of the target object;
   performing frequency-domain conversion on the time-domain signal to obtain a frequency-domain signal representing the physiological state of the target object; and
   determining, based on a peak value of the frequency-domain signal, the physiological state detection result of the target object.

6. The method of claim 5, wherein determining, based on the pixel brightness information of the three color channels of the region of interest, the time-domain signal representing the physiological state of the target object comprises:

   determining, based on the pixel brightness information of the three color channels corresponding to the region of interest, a time-domain brightness signal, which corresponds to each of the color channels, of the region of interest; and
   performing principal component analysis on the time-domain brightness signals, which correspond to the three different color channels, of the region of interest, to obtain the time-domain signal.

7. The method of claim 1 or 2, wherein the region attribute information comprises a region area, the to-be-extracted information comprises a weighted time-domain brightness signal, and performing, based on the region attribute information, the weighted processing or the region filtering on the at least one smooth region, to obtain the to-be-extracted information associated with the physiological state comprises:

   generating, for each of the at least one smooth region, a time-domain brightness signal corresponding to the smooth region, according to pixel brightness information of at least one color channel of the smooth region in the plurality of frames of face images; and

performing, based on an area of each of the at least one smooth region, the weighted processing on the time-domain brightness signal corresponding to the at least one smooth region, to obtain an area-weighted time-domain brightness signal;

wherein performing the physiological state information extraction on the to-be-extracted information to obtain the physiological state detection result of the target object comprises:

performing, based on the area-weighted time-domain brightness signal, the physiological state information extraction, to obtain the physiological state detection result.

8. The method of claim 7, wherein the smooth region corresponds to pixel brightness information of three different color channels, and generating, for the each of the at least one smooth region, the time-domain brightness signal corresponding to the smooth region, according to the pixel brightness information of the at least one color channel of the smooth region in the plurality of frames of face images comprises:

determining, based on the pixel brightness information, which corresponds to the three color channels, of the smooth region in the plurality of frames of face images, a time-domain brightness signal, which corresponds to each of the three color channels, of the smooth region;

wherein performing, based on the area of the each of the at least one smooth region, the weighted processing on the time-domain brightness signal corresponding to the at least one smooth region, to obtain the area-weighted time-domain brightness signal comprises:

performing, for each of the three color channels, the weighted processing on the time-domain brightness signal of each of the at least one smooth region in the color channel based on the area of the smooth region, to obtain the area-weighted time-domain brightness signal in the color channel;

wherein performing, based on the area-weighted time-domain brightness signal, the physiological state information extraction, to obtain the physiological state detection result comprises:

performing principal component analysis on the area-weighted time-domain brightness signal of the smooth region in a plurality of different color channels to obtain a time-domain signal representing the physiological state of the target object; and

performing frequency-domain processing on the time-domain signal representing the physiological state of the target object, and determining the physiological state detection result based on a peak value of a frequency-domain signal obtained by the frequency-domain processing.

9. The method of claim 1 or 2, wherein the region attribute information comprises an region area and region brightness, the to-be-extracted information comprises fused image information, and performing, based on the region attribute information, the weighted processing or the region filtering on the at least one smooth region, to obtain the to-be-extracted information associated with the physiological state comprises:

determining, according to an area of each of the at least one smooth region, a first contribution degree of the smooth region to the physiological state detection result;

determining, according to pixel brightness information of each of the at least one smooth region, a second contribution degree of the smooth region to the physiological state detection result; and

fusing, based on the first contribution degree and the second contribution degree, image information of the at least one smooth region, to obtain the fused image information;

wherein performing the physiological state information extraction on the to-be-extracted information to obtain the physiological state detection result of the target object comprises:

performing the physiological state information extraction on the fused image information to obtain the physiological state detection result.

10. The method of claim 9, wherein the first contribution degree comprises an area weight, the second contribution degree comprises a brightness weight, and fusing, based on the first contribution degree and the second contribution degree, the image information of the at least one smooth region, to obtain the fused image information comprises:

determining, based on a product of the area weight and the brightness weight corresponding to each of the at least one smooth region, a total weight value corresponding to the smooth region; and

performing, based on the corresponding total weight value, weighted fusing on the image information of the at least one smooth region, to determine the fused image information.

11. The method of claim 9, wherein the fused image information comprises a fused time-domain brightness signal, and fusing, based on the first contribution degree and the second contribution degree, the image information of the at least one smooth region, to obtain the fused image information comprises:

performing, for each of the at least one smooth region, time-domain processing on a brightness value of the smooth region in the plurality of frames of images, to determine a time-domain brightness signal corresponding to the smooth region; and
fusing, based on the first contribution degree and the second contribution degree, the time-domain brightness signals corresponding to a plurality of smooth regions, to obtain the fused time-domain brightness signal;
wherein performing the physiological state information extraction on the fused image information to obtain the physiological state detection result comprises:
performing frequency-domain processing on the fused time-domain brightness signal, and determining the physiological state result based on a peak value of a frequency-domain signal obtained by the frequency-domain processing.

12. The method of any one of claims 1 to 11, further comprising:

searching, based on the face image, body archive data matching the target object from a preset archive library;
performing, based on image information of the plurality of frames of face images, time-domain processing and frequency-domain processing, to obtain an estimated heart rate value of the target object; and
estimating, based on the body archive data and the estimated heart rate value, a blood pressure value of the target object, to obtain a blood pressure measurement result.

13. The method of claim 12, wherein searching, based on the face image, the body archive data matching the target object from the preset archive library comprises at least one of:

searching, based on face features of the target object extracted from the face image, body archive data corresponding to the face features of the target object from the preset archive library; or
searching, based on an identifier of the target object identified from the face image, body archive data corresponding to the identifier from the preset archive library.

14. The method of claim 12 or 13, wherein estimating, based on the body archive data and the estimated heart rate value, the blood pressure value of the target object, to obtain the blood pressure measurement result comprises:

determining an ejection time corresponding to the estimated heart rate value according to a linear fitting relationship between the ejection time and a heart rate; calculating a body surface area corresponding to the target object based on gender data, a height and a body weight comprised in the body archive data; and determining a heart elasticity value of the target object based on the body weight and an age comprised in the body archive data and the estimated heart rate value of the target object;
determining a stroke volume corresponding to the target object based on the ejection time, the body surface area, the age comprised in the body archive data, and the estimated heart rate value;
calculating an estimated pulse pressure value corresponding to the target object based on a ratio between the stroke volume corresponding to the target object and the heart elasticity value; and
calculating a systolic pressure value and a diastolic pressure value of the target object based on the estimated pulse pressure value corresponding to the target object and the gender data comprised in the body archive data.

15. The method of any one of claims 1 to 14, wherein the video stream comprises a video stream in a vehicle cabin, and extracting the plurality of frames of face images of the target object from the plurality of frames of images in the video stream comprises:
determining, according to a face detection result of the video stream and a designated riding position, the face image of the target object from detected face images, wherein the designated riding position is used to indicate a position of the target object which is being measured.

16. The method of claim 15, further comprising at least one of:

transmitting the physiological state detection result of the target object to a display screen in the vehicle cabin, to display the physiological state detection result on the display screen; or
transmitting the physiological state detection result of the target object to a server side of a physiological state measurement application, to send the physiological state detection result through the server side to a terminal device used by the target object in case that the target object requests to obtain the detection result through the physiological state application.

17. The method of any one of claims 1 to 16, further comprising:
performing following operations repeatedly in case that a new video stream is obtained, until a preset detection duration is reached and an updated physiological state detection result is obtained:
extracting the plurality of frames of face images of the target object from the new video stream; determining the at least one smooth region in each frame of face image; performing, based on the region attribute information, the weighted processing or the region filtering on the at least one smooth region, to obtain the to-be-extracted information associated with the physiological state; and updating the physiological state detection result based on the to-be-extracted information.

18. The method of claim 17, further comprising:
generating a detection process reminder signal according to a duration of the obtained video stream and the preset detection duration, wherein the detection process reminder signal is used to remind the target object of a required detection duration.

19. A physiological state detection apparatus, comprising:

an obtaining part, configured to obtain a video stream collected by a camera device;
an extraction part, configured to extract a plurality of frames of face images of a target object from a plurality of frames of images in the video stream;
a determination part, configured to determine at least one smooth region in the face image;
a processing part, configured to perform, based on region attribute information, weighted processing or region filtering on the at least one smooth region, to obtain to-be-extracted information associated with a physiological state; and
a detection part, configured to perform physiological state information extraction on the to-be-extracted information to obtain a physiological state detection result of the target object.

20. An electronic device, comprising a processor, a memory and a bus, wherein the memory has stored thereon machine-readable instructions executable by the processor, the processor and the memory communicate through the bus when the electronic device is in operation, and the machine-readable instructions, when executed by the processor, perform the physiological state detection method of any one of claims 1 to 18.

21. A computer-readable storage medium having stored thereon a computer program that when run by a processor, performs the physiological state detection method of any one of claims 1 to 18.

22. A computer program comprising computer-readable codes, wherein in case that the computer-readable codes are run in an electronic device, a processor of the electronic device performs the physiological state detection method of any one of claims 1 to 18.

A video stream collected by a camera device is obtained — S101

↓

Multiple frames of face images of a target object are extracted from multiple frames of images in the video stream — S102

↓

At least one smooth region in the face image is determined — S103

↓

Weighted processing or region filtering is performed on the at least one smooth region based on region attribute information, to obtain to-be-extracted information associated with a physiological state — S104

↓

Physiological state information extraction is performed on the to-be-extracted information to obtain a physiological state detection result of the target object — S105

**FIG. 1**

**FIG. 2A**

A video stream collected by a camera device is obtained — S101

Multiple frames of face images of a target object are extracted from multiple frames of images in the video stream — S102

At least one smooth region in the face image is determined — S103

At least one region of interest in which the region brightness satisfies a preset condition is determined from the at least one smooth region — S201

The physiological state information extraction is performed on the image information of the at least one region of interest to obtain the physiological state detection result — S202

**FIG. 2B**

A video stream collected by a camera device is obtained — S101

Multiple frames of face images of a target object are extracted from multiple frames of images in the video stream — S102

At least one smooth region in the face image is determined — S103

For each smooth region, a time-domain brightness signal corresponding to the smooth region is generated according to the pixel brightness information of at least one color channel of the smooth region in the multiple frames of face images — S203

The weighted processing is performed on the time-domain brightness signal corresponding to the at least one smooth region based on the area of each smooth region, to obtain an area-weighted time-domain brightness signal — S204

The physiological state information extraction is performed based on the area-weighted time-domain brightness signal, to obtain the physiological state detection result — S205

**FIG. 2C**

A video stream collected by a camera device is obtained — S101

Multiple frames of face images of a target object are extracted from multiple frames of images in the video stream — S102

At least one smooth region in the face image is determined — S103

The first contribution degree of each smooth region to the physiological state detection result is determined according to the area of the smooth region — S206

The second contribution degree of each smooth region to the physiological state detection result is determined according to the pixel brightness information of the smooth region — S207

The image information of the at least one smooth region is fused based on the first contribution degree and the second contribution degree, to obtain the fused image information — S208

The physiological state information extraction is performed on the fused image information to obtain the physiological state detection result — S209

**FIG. 2D**

```
                                                                          S101
┌─────────────────────────────────────────────────────────────────┐  ⌐
│          A video stream collected by a camera device is obtained  │ ⌐
└─────────────────────────────────────────────────────────────────┘
                                  │
                                  ▼                                       S102
┌─────────────────────────────────────────────────────────────────┐  ⌐
│      Multiple frames of face images of a target object are extracted from │
│              multiple frames of images in the video stream        │
└─────────────────────────────────────────────────────────────────┘
                                  │
                                  ▼                                       S210
┌─────────────────────────────────────────────────────────────────┐  ⌐
│   Body archive data matching the target object is searched from a preset │
│                 archive library based on the face image           │
└─────────────────────────────────────────────────────────────────┘
                                  │
                                  ▼                                       S211
┌─────────────────────────────────────────────────────────────────┐  ⌐
│  Time-domain processing and frequency-domain processing are performed │
│  based on the image information of the multiple frames of face images, to │
│             obtain an estimated heart rate value of the target object │
└─────────────────────────────────────────────────────────────────┘
                                  │
                                  ▼                                       S212
┌─────────────────────────────────────────────────────────────────┐  ⌐
│  A blood pressure value of the target object is estimated based on the body │
│  archive data and the estimated heart rate value, to obtain a blood pressure │
│                         measurement result               │
└─────────────────────────────────────────────────────────────────┘
```

**FIG. 2E**

Obtaining Part 301

Extraction Part 302

Determination Part 303

Processing Part 304

Detection Part 305

**FIG. 3**

**FIG. 4**

## INTERNATIONAL SEARCH REPORT

International application No.

**PCT/CN2022/113755**

**A. CLASSIFICATION OF SUBJECT MATTER**

G06V 40/70(2022.01)i;  G06V 40/16(2022.01)i

According to International Patent Classification (IPC) or to both national classification and IPC

**B. FIELDS SEARCHED**

Minimum documentation searched (classification system followed by classification symbols)

G06V

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 视频, 图像, 图片, 生理, 心率, 心跳, 血压, 血氧, 呼吸, 健康, 平滑区域, 平坦区域, 脸颊, 面颊, 额头, 前额, 权重, 加权, 融合, 时域, 频域, 面积, 身份, video, image, photo, picture, physiology, heart w rate, heartbeat, blood w pressure, oximeter, breath+, health, smooth w region, flat w region, cheek, forehead, weight, fusion, time w domain, frequency w domain, measure, identity

**C. DOCUMENTS CONSIDERED TO BE RELEVANT**

| Category* | Citation of document, with indication, where appropriate, of the relevant passages | Relevant to claim No. |
|---|---|---|
| PX | CN 114648749 A (SHANGHAI SENSETIME LINGANG INTELLIGENT TECHNOLOGY CO., LTD.) 21 June 2022 (2022-06-21)<br>description, paragraphs 0069-0181 | 1-6, 15-22 |
| PX | CN 114863399 A (SHANGHAI SENSETIME LINGANG INTELLIGENT TECHNOLOGY CO., LTD.) 05 August 2022 (2022-08-05)<br>description, paragraphs 0067-0172 | 1-2, 7-8, 15-22 |
| PX | CN 114663865 A (SHANGHAI SENSETIME LINGANG INTELLIGENT TECHNOLOGY CO., LTD.) 24 June 2022 (2022-06-24)<br>description, paragraphs 0067-0190 | 1-3, 9-11, 15-22 |
| PY | CN 114708225 A (SHANGHAI SENSETIME LINGANG INTELLIGENT TECHNOLOGY CO., LTD.) 05 July 2022 (2022-07-05)<br>description, paragraphs 0068-0182 | 12-14 |
| Y | CN 111275018 A (EAST CHINA NORMAL UNIVERSITY) 12 June 2020 (2020-06-12)<br>description, paragraphs 0039-0083 | 1-6, 12-22 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 November 2022** | **29 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2022/113755** |

### C. DOCUMENTS CONSIDERED TO BE RELEVANT

| Category* | Citation of document, with indication, where appropriate, of the relevant passages | Relevant to claim No. |
|---|---|---|
| Y | CN 112819790 A (NANJING UNIVERSITY OF POSTS AND TELECOMMUNICATIONS) 18 May 2021 (2021-05-18) description, paragraphs 0042-0096 | 1-6, 12-22 |
| A | CN 113197558 A (CENTRAL SOUTH UNIVERSITY) 03 August 2021 (2021-08-03) entire document | 1-22 |
| A | CN 106236049 A (NANJING INSTITUTE OF TECHNOLOGY) 21 December 2016 (2016-12-21) entire document | 1-22 |
| A | CN 113693573 A (XIDIAN UNIVERSITY) 26 November 2021 (2021-11-26) entire document | 1-22 |
| A | JP 2018164587 A (NEC CORPORATION) 25 October 2018 (2018-10-25) entire document | 1-22 |
| A | WO 2018179150 A1 (NEC CORPORATION) 04 October 2018 (2018-10-04) entire document | 1-22 |

Form PCT/ISA/210 (second sheet) (January 2015)

INTERNATIONAL SEARCH REPORT
Information on patent family members

| International application No. |
| --- |
| **PCT/CN2022/113755** |

| Patent document cited in search report | | | Publication date (day/month/year) | Patent family member(s) | | | Publication date (day/month/year) |
| --- | --- | --- | --- | --- | --- | --- | --- |
| CN | 114648749 | A | 21 June 2022 | None | | | |
| CN | 114863399 | A | 05 August 2022 | None | | | |
| CN | 114663865 | A | 24 June 2022 | None | | | |
| CN | 114708225 | A | 05 July 2022 | None | | | |
| CN | 111275018 | A | 12 June 2020 | None | | | |
| CN | 112819790 | A | 18 May 2021 | None | | | |
| CN | 113197558 | A | 03 August 2021 | None | | | |
| CN | 106236049 | A | 21 December 2016 | None | | | |
| CN | 113693573 | A | 26 November 2021 | None | | | |
| JP | 2018164587 | A | 25 October 2018 | None | | | |
| WO | 2018179150 | A1 | 04 October 2018 | JP | WO2018179150 | A1 | 07 November 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210344726
- CN 202210344718
- CN 202210346427
- CN 202210346417